# EUROPEAN PATENT APPLICATION

(11) **EP 4 461 273 A1**
(43) Date of publication of application: **13.11.2024**
(21) Application number: 23172566.4
(22) Date of filing: 10.05.2023
(51) Int. Cl.: A61F 5/01

(54) **A SOFT, SLIDING MECHANISM FOR FINGER EXTENSION**

(71) Applicant: Universität Heidelberg, 69117 Heidelberg (DE); Morgalla, Maximilian, 69214 Eppelheim (DE)
(72) Inventor: MORGALLA, Maximilian, 69214 Eppelheim (DE); ALICEA, Ryan, 69115 Heidelberg (DE); MASIA, Lorenzo, 69121 Heidelberg (DE)
(74) Representative: Müller-Boré & Partner Patentanwälte PartG mbB

(57) **Abstract**

The invention relates to a user wearable hand orthosis comprising:
a support structure (100) comprising a dorsal support portion (110) configured to be worn on at least a part of a back of the hand of the user, the dorsal support portion (110) having a first surface on a dorsal side of the dorsal support portion (110) and a second surface on a side of the dorsal support portion (110) opposite to the dorsal side;
a sliding mechanism comprising a sliding element (210) configured to be movable along a track on the second surface side of the dorsal support portion (110) between a first end position and a second end position, and
at least one finger module (300) configured to be worn on at least one finger of the hand of the user, each of the at least one finger modules (300) comprising a distal end portion (310) configured to be worn on at least one distal section of the at least one finger, and a sliding element connection portion (340), configured to be connectable to the sliding element (210).

The invention further relates to a method of manufacturing a user wearable hand orthosis.

## Description

The invention relates to a user wearable hand orthosis and a method of manufacturing a user wearable hand orthosis.

The invention lies in the field of user wearable orthoses, and in particular in the field of tendon-driven wearable glove orthoses. Specifically, members of a population suffering from some form of, for example, upper body paralysis from a stroke, spinal cord injury, brachial plexus injury, or other causes may struggle to perform certain movements, such as grasping movements normally executed by a hand. To aid in performing such movements or restoring the ability to perform said movements, different types of mechanical devices have previously been developed.

Exemplary mechanical devices include tendon-driven powered glove devices, wherein a tendon connected to the dorsal tip of the finger pulls, when under tension, the finger into extension (see e.g., the publications Xiloyannis, Michele, et al., "Modelling and design of a synergy-based actuator for a tendon-driven soft robotic glove", 2016, 6th IEEE International Conference on Biomedical Robotics and Biomechatronics (BioRob), Yurkewich, Aaron, et al. "Hand Extension Robot Orthosis (HERO) Glove Development and Testing With Stroke Survivors With Severe Hand Impairment", 2019, IEEE Transactions on Neural Systems and Rehabilitation Engineering", 27.5 (2019): 916-926 and Kang, Brian Byunghyun, et al. "Development of a polymer-based tendon-driven wearable robotic hand", 2016 IEEE International Conference on Robotics and Automation (ICRA), IEEE, 2016.

Another known tendon-driven solution is to install a rigid structure on the back of the hand to which a tendon can be attached. The tendon, attached on the underside of the structure (the dorsal side of the hand) causes the structure to curl when the tendon is under tension which pushes the hand closed.

Direct tendon-driven systems have, however, problems which arise from safety concerns due to hyperextension of the fingers and comfort concerns from tendon tension on back of the hand. Thus, extra considerations, whether mechanical or electrical, must be made to ensure that hyperextension of the fingers does not occur Also, because of the positioning of the tendons, they cut into the back of the fingers when the tendons are under tension. This can result m discomfort and even pain. Back of the hand tendon driven systems typically add a large bulk and mass to the back of the hand. Further, these systems have high transmission losses due to the mechanism structure which often results m weak grasping force.

Other mechanical devices involve passive mechanisms for extending the fingers using either springs or other elastic elements. One drawback of the known passive solutions is that these perform sufficiently well only when the tension on the tendons for flexion is released. This is, however, possible only on a relaxed healthy person's hand when muscles tension is consciously released. Typically, passive mechanisms are, therefore, unable to provide enough force for patients with a spastic hand. For such patients, in addition to the force of moving the fingers an additional force of overcoming the spastic muscles is required as they are actively working against the opening motion,

It is therefore an object of the present invention to provide a user wearable hand orthosis and a method for manufacturing a user wearable hand orthosis that overcome one or more of the above disadvantages. In particular, it is an object of the present invention to provide a user wearable hand orthosis with improved operational characteristics and user adaptability. This object is solved by a user wearable hand orthosis and a method for manufacturing a user wearable hand orthosis having the features as recited in the independent claims. Preferred embodiments form the subject of the dependent claims.

The invention is based on an observation made during extensive research carried out by the inventors that while the palm of the hand can morph and shapeshift to be able to wrap around any object and to securely grasp it, a major part of the back of the hand stays approximately flat during the grasping motion. The user wearable hand orthosis of the aspect described below advantageously utilizes this area for a sliding motion such as a linear sliding motion, in order to coerce the fingers into an extended position.

Thus, aspect of the disclosure relates to a user wearable hand orthosis, such as a tendon-driven hand orthosis and/or a glove orthosis, preferentially a tendon-driven glove orthosis. In particular, while the following description may be given in terms of tendon-driven glove orthoses for explanatory purposes, it is understood that other types of user wearable orthoses may, *mutatis mutandis,* similarly be implemented, such as for example hydraulic driven hand orthosis.

The user wearable hand orthosis may be configured to be worn by a user, wherein the user wearable hand orthosis may be configured to be mountable at least partially on a hand of the user. Mountable at least partially on the hand of the user may be understood in this context as mountable on the hand of the user, wherein at least some components of the user wearable hand orthosis may be mountable on other parts of the user, such as for example on an arm, e.g., a lower arm, and/or a torso of the user, e.g., in a shoulder region of the user. As an example, an actuator connected to at least one tendon connectable to the user wearable hand orthosis may be located and/or worn on a torso or hip of the user.

The user wearable hand orthosis may comprise a support structure (hand support structure) comprising a dorsal support portion configured to be worn on at least a part of a back of the hand of the user. The dorsal support portion has a first surface on a dorsal side (i.e., the side facing the back of the hand) of the dorsal support portion and a second surface on a side of the dorsal support portion opposite to the dorsal side (i.e., the side facing away from the back of the hand). In other words, the second surface may be a surface opposite to the first surface.

The user wearable hand orthosis may further comprise a sliding mechanism comprising a sliding element (slider) configured to move (for example slidably move or slide) along a track on the second surface side of the dorsal support portion. Moving along a track on the second surface side of the dorsal support portion encompasses moving along a predetermined path above or in slidable contact with the second surface of the dorsal support portion and/or moving along a track provided by at least one guiding element (e.g., at least one guiding rail, guiding recess or other guiding element) provided on and/or connected to the second surface of the dorsal support portion. The guiding element may be a separate element or may be an integral part of the dorsal support portion and specifically an integral part of the second surface of the dorsal support portion.

In particular, the sliding element may be configured to be movable or move between a first end position and a second end position, wherein in a use state of the hand orthosis, the first end position is arranged proximal to a wrist portion of the hand of the user (for example may be arranged substantially over a wrist joint portion of the hand) and the second end position is arranged at a distance from the first end position in a direction toward a knuckle joint portion (i.e., direction toward the fingers) of the hand of the user. Thus, the distance of the second end position from a first end portion (e.g., wrist portion) of the dorsal support portion is greater than a distance of the first end position from the first end portion of the dorsal support portion. In other words, the sliding element may be movable along a track in a substantially longitudinal direction of the user's hand in a use state of the user wearable hand orthosis. The track may be a substantially linear track. The use state (as worn state) of the user wearable hand orthosis is a state of the user wearable hand orthosis in which the user wearable hand orthosis is mounted on a hand of the user.

The user wearable hand orthosis may further comprise at least one finger module configured to be worn on at least one finger of the hand of the user, wherein each of the at least one finger module comprises a distal end portion configured to be worn on at least one distal section of the at least one finger and a sliding element connection portion configured to be connectable or be connected to the sliding element. The distal end portion may be arranged on one end of the finger module (distal end of the finger module) and the sliding element connection portion may be arranged on the other end of the finger module (proximal end of the finger module). Connecting two elements, sections, structures, portions, etc. within the scope of the disclosure encompasses a direct connection and indirect connection (e.g., through intermediate elements, layers, etc),

By the provision of a sliding mechanism comprising a sliding element configured to be movable (for example to be slidably movable or slide) along a track on the second surface side of the dorsal support portion, it is possible to advantageously utilize the area on the back of the hand which stays approximately flat during the grasping motion.

Having a sliding feature allows further to easily connect at least one, preferably multiple fingers to the same sliding element. This sliding element can then be actuated by a single tendon and single motor or other actuator (one-to-many relationship). For the extension of the fingers this is sufficient, as the control of extension doesn't have to be as granular as the flexion of the fingers. While it might be desirable to be able to flex individual fingers to be able to execute a fine pinch grasp or all fingers for a cylindrical grasp, for the extension of the fingers, typically the most desirable or useful feature is to be able to open whichever fingers have been previously closed.

Accordingly, it is possible to realize a compact and lightweight mechanism for finger extension, which has reduced mechanical complexity and is easy to operate by the user, while generating sufficient hand extension force to assist grasping movements. In an example, the sliding mechanism may comprise one sliding element, thus further reducing the mechanical complexity of the user wearable hand orthosis. It is, however, possible to provide a plurality of sliding elements, for example for movement of an individual finger or a plurality of fingers. Thus, it might be possible to realize a mechanism for finger extension that is more versatile.

Further, by employing a sliding mechanism that provides an attachment point for at least one finger onto the back of the hand, it is possible to configure the sliding mechanism to be substantially flat and low-profile. Since the length of the back of the hand naturally restricts the movement of the sliding element, it is possible to easily limit the stroke length of the motion and thus prevent hyperextension of the at least one finger. Still further, because the tension is applied to the finger module, instead of the finger itself, discomfort from wire tension can be eliminated or substantially reduced. Still further, the support structure (such as a glove structure) can be easily turned into a type of a Bowden tube, thereby efficiently transmitting the mechanical force (e.g., pulling force) to at least one finger of the user.

The support structure may be for example a glove structure, with one or more portions or sections made of fabric, elastic polymer and/or other flexible materials. A combination of different materials is also possible. Advantages of a glove structure include at least one of lightweight, adaptable to various hands and easy to don and doff. Further, advantages may include better joint alignment, it allows for a close fit to the hand and thus reduces the problems associated with joint alignment. However, the support structure is not limited to a glove structure and other structures (such as for example a rigid structure or a combination of a rigid and a glove structure) may be realized.

The support structure may have a dorsal support portion as described above. In a use state the dorsal support portion may exhibit a form that substantially conforms to the form of the back portion of the hand. Slight deviations in this form are also possible.

The user wearable hand orthosis may comprise further support portions, such as a palmar support portion configured to be worn on at least a part of a palm of the hand of the user and/or a wrist portion configured to be worn on at least a part of the wrist of the user. In a use state of the user wearable hand orthosis the palmar support portion may be exhibit a form that substantially conforms to the form of the palmar portion of the hand. Similarly, in a use state of the user wearable hand orthosis the wrist portion may exhibit a form that substantially conforms to the wrist of the hand. For example, the wrist portion may be configured to be wrapped around the wrist of the hand of the user and may comprise or consist of one or more bands (wrist bands) that may be wrapped around the wrist of the user. The wrist support portion may further comprise a locking section (e.g., Velcro, etc.) configured to secure the wrist support portion in its wrapped position around the wrist of the user.

The dorsal support portion and/or the palmar support portion and/or the wrist portion may be formed integrally or may be formed as separate modules or components that may be fixedly or releasably connectable to one another, thereby forming a support structure to be worn or arranged on the hand of a user. Further, a combination of integral forming, fixed (i.e., permanent) and/or releasable connection is also possible, wherein some parts of the dorsal support portion and/or the palmar support portion and/or the wrist portion may be integrally formed or fixedly connected and some parts of the dorsal support portion and/or the palmar support portion and/or wrist portion may be releasably connectable by the use of locking sections, fasteners, etc.

To realize the connection, the dorsal support portion and the palmar support portion and optionally the wrist portion may be provided with a respective at least one locking section, wherein the locking sections of the dorsal support portion, the palmar support portion and optionally the wrist portion are configured to be engageable with each other. The support structure may be configured to be wrapped substantially around the hand of the user to bring the locking sections of the dorsal support portion, the palmar support portion and optionally the wrist portion into engagement with each other to mount the support structure on the hand of the user. The locking sections may be configured as hook-loop type locking sections, Velcro type locking sections, clamps, etc.

The support structure may be configured to be substantially planar when not worn by the user and may be arrangeable on the hand and optionally the wrist of the user, in order to mount the support structure on the hand of the user. For example, the support structure may be wrappable around the hand and optionally the wrist of the user. This may, on one hand, allow easy and safe storage of the support structure when not in use, and, on the other hand, simplify designing and/or manufacturing of the support structure (e.g., additional hardware may be more easily mountable on a planar support structure).

The support structure, in particular one or more of the dorsal support portion, the palmar support portion and the wrist portion may comprise various zones or sections. For example, the support structure (and in particular one or more of the aforementioned portions) may comprise at least one flex zone or section configured to flex to allow the support structure to be wrapped and/or bent around the hand of the user. Other zones or sections may have lower flexibility and/or higher stiffness that the flex zone or section. For example, the support structure and in particular the dorsal support portion, the palmar support portion and/or the wrist portion may comprise one or more compression load absorption zones or sections configured to at least partially absorb compression load within the support structure. Further, as described above, the support structure (and in particular one or more of the aforementioned portions) may comprise one or more locking sections or zones.

The user wearable hand orthosis further comprises one or more finger modules (also referred to as "end effector"). Each finger module is configured to be worn on at least one finger of the user, such as an index finger, a middle finger, a ring finger, and/or a little finger. For example, each finger module may be configured to be worn on one finger of the user. At least one finger module may also be configured to be worn on more than one finger, such as on two, three or all of the index finger, a middle finger, a ring finger, and a little finger.

The one or more finger modules, preferably each finger module may be configured to be worn at least on a distal section, e.g., on a distal phalanx section, of the respective at least one finger. For example, the one or more finger modules, preferably each finger module may comprise a distal end portion that is configured to at least partially enclose a distal section, e.g., a distal phalanx section of the respective at least one finger of the user. In particular, the distal end portion of the finger module may be configured such that there is at least one contact point between the distal end portion of the finger module and the tip, in particular the front portion of the tip, of the at least one finger. The distal end portion may be configured in form of a cap that may be worn over the tip of the at least one finger and may at least partially enclose the at least one distal section and in particular the tip of the at least one finger. Most of the spastic forces in the hand may lie in one or more distal and/or middle joints of the fingers with comparatively low resistance in the proximal (metacarpal) joints. Therefore, configuring said one or more finger modules to be worn on the at least distal section and in particular over a fingertip allows a more effective force transfer and response to spastic resistance.

The at least one finger module may be configured to be connectable or be connected to the sliding element of the user wearable hand orthosis. The connection may be in particular a rigid connection, in order to ensure good force transfer. To realise the connection, the sliding element and the at least one finger module may comprise respective connection portions and/or connection elements. For example, the sliding element may comprise a finger module connection portion for connection of the one or more finger modules. If there is a plurality of finger modules connected to or connectable to the sliding element, the sliding element may comprise a plurality of finger module connection portions for individual connection of a respective one of a plurality of finger modules. The at least one finger module, preferentially each finger module may comprise a corresponding sliding element connection portion for connection with the sliding element (in particular for connection with at least one finger module connection portion of the sliding element).

The connection of the at least one finger module to the sliding element may be a fixed connection or a releasable connection. A fixed connection may allow a very secure connection between the respective finger module(s) and the sliding mechanism. Furthermore, a fit of the connection may be determined during and/or before manufacture and/or fitting of the support structure and/or the sliding mechanism, and may therefore no longer need to be checked during mounting thereof on the user and/or during the use of the user wearable hand orthosis. Alternatively, at least one finger module may be, preferentially releasably, connectable with and/or fixable to the sliding element. In other words, at least one finger module may be detachable. This may allow a significantly facilitated mounting of the support structure and the finger modules on the user, such as for users suffering from hand spasticity. Furthermore, the hand orthosis may be adapted to specific requirements of the user during the mounting thereof on the user. For example, it is possible to allow for a length adjustment based on different hand sizes. This may allow improved use flexibility, wherein a given hand orthosis may easily be adapted to multiple different users. A combination of fixed and releasable connection (for example for the different fingers) is also possible.

Not all of the finger modules need to be connected or to be connectable to the sliding element. For example, the hand orthosis may comprise at least one finger module that is configured to be fixedly or releasable connectable or be connected to the support structure. For example, the support structure may comprise at least one finger module connection section, wherein at least one other finger module may be connectable, preferentially releasably connectable, to the at least one finger module connection section. As described above, a fixed connection may be very secure and may be adjustable during the manufacturing of the hand orthosis. A releasable connection may significantly ease the mounting of the support structure and the finger modules on the user, such as for users suffering from hand spasticity and may allow adaptation to individual user requirements or hand anatomy.

The user wearable hand orthosis may further comprise a thumb module. The thumb module may be configured to be worn on a thumb of the user. The thumb module may be integrally formed with the dorsal and/or palmar support portions and/or wrist portion. Alternatively, the thumb module may be connectable with and/or fixable to the support structure and in particular to one or more of the dorsal support portion, palmar support portion and wrist portion. The thumb module may be configured similarly to the finger module described herein. The thumb module may be configured to be worn at least on a distal section, e.g., on a distal phalanx section, of the thumb of the user. Thereby, a more effective response to spastic resistance in the thumb of the user may be enabled.

At least one of, preferably each, dorsal support portion, palmar support portion, wrist portion, sliding mechanism, finger module(s), and/or the thumb module may be dimensioned according to the specific requirements of the user, such as for example to provide a good fit to the hand of the user. Furthermore, as described above, at least one of, preferably each, dorsal support portion, palmar support portion, wrist portion, sliding mechanism, finger module(s), and/or the thumb module may be configured to be separable from one another. In particular, it may therefore be possible to significantly improve an ease of use of the user wearable hand orthosis, such as for example during mounting and/or dismounting of the user wearable hand orthosis onto/from the hand of the user. In addition, a replaceability of at least one, preferably each, finger module, hand body sub-structure, palm sub-structure, back sub-structure, sliding mechanism, wrist module and/or the thumb module may therefore be also improved.

It is also possible that at least one of the finger modules and/or the thumb module may be integrally formed with the sliding element and/or at least one of the portions of the support structure (such as the dorsal and/or the palmar support portion), while at least one other of the one or more other finger module and/or the thumb module may be connectable with and/or fixable to the sliding element and/or at least one of the portions of the support structure. For example, a finger module configured to be worn on a little finger of the user may be integrally formed with or may be releasably connectable to the dorsal support portion, while other finger modules configured to be worn on the other fingers of the hand of the user (e.g., the index finger, middle finger, and/or ring finger) may be fixedly or releasably connectable with the sliding element. Another example is a thumb module that may be integrally formed with the dorsal support portion and/or palmar support portion, while one or more finger module configured to be worn on one or more fingers of the hand of the user may be releasably connectable with and/or releasably fixable to the sliding element and/or the dorsal support portion. In particular, mounting the user wearable hand orthosis on the user may thereby be facilitated, while still allowing a high degree of adaptability.

However, the present disclosure is not limited to the above configurations, and other configurations may be possible.

As described above, the user wearable hand orthosis may further comprise a sliding mechanism having a sliding element configured to be movable (e.g., slidably movable) along a track between a first end position and a second end position.

The motion (e.g., sliding motion of the sliding element may change the distance or length between an end portion of the dorsal support portion (e.g., an end portion that is arranged proximal to the wrist of the hand of the user) and the anchor/contact point or points of the at least one finger module (and thus the proximal end of the at least one finger module). Thus, a pulling force may be exerted on the fingers of the user's hand forcing the hand into an opened (extended) position.

To enable movement of the sliding element, the user wearable hand orthosis may further comprise at least one force transfer element connectable to the sliding element and configured to exert a (pulling) force on the sliding element, such as to enable the movement of the sliding element between the first end position and the second end position.

The sliding element may respectively comprise a force transfer element connection portion configured to enable connection of the at least one force transfer element with the sliding element.

The force transfer element may be connected to an actuator (that may be a part of the user wearable hand orthosis), wherein the actuator is configured to exert a force, such as a (pulling) force, on the at least one force transfer element and thus on the sliding element. By exerting a (pulling) force on the at least one force transfer element connected to the sliding element of the sliding mechanism, the user wearable hand orthosis may be configured to assist a movement, such as a grasping movement, of the hand of the user.

In an example, the user wearable hand orthosis may comprise a single force transfer element connected or connectable to the sliding element, thus realizing one-to-many connection (via the sliding element) between the actuator and the at least one finger module connected to the sliding element. Thus, the force transfer mechanism may be simplified and the weight of the user wearable hand orthosis that acts on the hand reduced. At the same time, may be possible to efficiently aid or enable a grasping movement of the hand.

The connection of the at least one force transfer element to the sliding mechanism and/or the actuator may be a fixed connection or a releasable connection. A releasable connection may ease the mounting of the user wearable hand orthosis on user's body as well as the cleaning of the user wearable hand orthosis.

The user wearable hand orthosis may also comprise at least one additional force transfer element. The at least one additional force transfer element may be configured to transfer force (such as pulling force) to each individual finger, to cause a finger movement, such as a bending movement of at least one finger joint or a plurality of finger joints. This aids or enables more complex grasping or finger moving patterns. The additional force transfer elements may for example extend along or be integrated into the palmar support portion.

The at least one force transfer element may be a tendon (such as an artificial tendon). The term "tendon" is intended to be interpreted broadly herein and encompasses artificial tendons, cables (such as Bowden cables comprising an outer sheath and one or more inner cables positioned inside the outer sheath), straps (such as high tensile strength fabric straps), belts (such as elastomer belts), chains, strings, ribbons, etc. A combination of one or more different types of force transfer elements is also possible.

The user wearable hand orthosis may comprise at least one force transfer element guide element, configured to guide at least one force transfer element (e.g., a tendon) along a given path to the sliding element and/or finger module and/or thumb module, etc. Guiding at least one force transfer element may be understood as at least partially restricting at least one degree of freedom of movement of the at least one force transfer element with respect to the support structure. For example, guiding at least one force transfer element may be understood as defining at least one force transfer element routing path (preferentially one force transfer element routing path for each force transfer element) along the support structure (for example along one or more of the dorsal support portion, palmar support portion and wrist portion), wherein the at least one force transfer element may be moveable along the at least one force transfer element routing path while being restricted by the force transfer element guide elements to said at least one force transfer element routing path.

The at least one force transfer element guide element may be made from the same material as a core layer (described in further detail below) of the support structure. The at least one force transfer element guide element may be made from other material, such as Teflon° PTFE. The at least one force transfer element guide element may have an at least partially hoop-like and/or tube-like shape, wherein the at least one force transfer element may be guided within a central cavity or opening of the at least one force transfer element guide element. The at least one force transfer element guide element may be 3D printable onto and/or integrally formed with a core layer of the support structure. One or more of the at least one force transfer element guide elements may define a routing path for each of the at least one force transfer elements.

Upon the exertion of a force (such as a pulling force), the sliding element moves along a track on the surface side of the dorsal support portion, which is opposite to the dorsal side of the dorsal support portion. In an example, the sliding mechanism comprises further at least one guiding element, wherein the sliding element is movably mountable on the at least one guiding element. The at least one guiding element thus defines the track along which the sliding element moves, when a force (e.g., pulling force) is exerted on the sliding element via the at least one force transfer element.

The at least one guiding element may be or may comprise at least one guiding rail arranged on the second surface of the dorsal support portion, wherein the sliding element is movably mounted or mountable on the guiding rail. The at least one guiding rail may have an elongated, substantially linear form and may have a cross-section in a thickness direction having a substantially rectangular form (with or without rounded edges), oval form or any other suitable form. The dimensions (width, thickness, length) may be suitably selected depending on the material and/or the sizes of the hand. Exemplary and non-limiting dimensions are: width in the range of about 3 mm to 15 mm, more specifically about 4 mm to 12 mm, further specifically about 5 mm to 7 mm; length in the range of about 30 mm to 80 mm, more specifically about 40 mm to 70 mm, further specifically about 45 mm to 60 mm, thickness (height) in the range of about 0.1 mm to 1 mm, more specifically about 0.2 mm to 0.8 mm further specifically about 0.3 mm to 0.5 mm. Exemplary guiding rail sizes are 6 x 50 x 0.4 mm or 6 x 50 x 0.3 mm. Deviations are, however, also possible depending, for example, on the intended use of the user wearable hand orthosis.

The at least one guiding rail may be connected to the dorsal support portion and may be arranged on the surface (second surface) of the dorsal support portion that is on the side opposite to the dorsal side. The connection may be a fixed connection. For example, the at least one guiding rail may be made integral with the dorsal support portion or may be fixedly secured to the dorsal support portion by suitable means, such as adhesive. It is also possible to releasably connect the at least one guiding rail to the dorsal support portion. For example, the dorsal portion and specifically the second surface of the dorsal support portion may be provided with at least one guiding rail fastening (connecting) portion configured to accept and securely hold in place the at least one guiding rail.

Alternatively or in addition, the guiding element may be or may comprise at least one guiding recess or groove, which is configured to be engageable with a corresponding projection (e.g., a rail, etc.) provided on the dorsal side of the sliding element. The at least one guiding recess or groove may, for example, be formed on the second surface of the dorsal support portion, i.e., on the surface of the dorsal support portion that is on the side facing away from the back of the hand of the user. The at least one guiding recess or groove may define the track along which the sliding element moves upon exertion of force (pulling force) via the force transfer element (such as e.g., tendon).

In an example, a plurality (i.e., two or more) of guiding elements, such as guiding rails, guiding recessed, guiding grooves, etc., may be provided. A combination of different types of guiding elements (such as guiding rail(s) and guiding recess(es) or guiding groove(s)) is also possible.

The at least one guiding element (e.g., guiding rail, recess or groove) may comprise at least one portion made of a low friction material. The at least one portion made of low friction material may be the portion which engages with (i.e., which comes into contact with) the sliding element. This portion may comprise with a layer of low friction material or may be made of a low friction material. It is also possible to form the entire guiding element of low friction material.

Low friction material may be understood within the scope of the disclosure unless individually specified as a material that exhibits a coefficient of friction in kinematic and/or static conditions equal or lower than 0.4, for example equal or lower than 0.35. For example, the coefficient of friction in kinematic and/or static conditions may be in the range of about 0.04 to 0.1. Non-limiting examples of low friction material are Teflon° PTFE, the iGlidur^{®} material family, Nylon 6/6, some types of hardened stainless steel, etc.

The sliding element may be formed in a suitable size, form and/or of material . For example, the sliding element may be made of or may comprise any suitable material, such as hardened stainless steel, spring steel (e.g., low-alloy manganese or medium-carbon steel or high-carbon steel), polymer (such as elastic polymer), a material from the iGlidur^{®} material family, or any other suitable material.

The sliding element may comprise a body portion having a substantially plate-like form, e.g., made of the above mentioned materials. Substantially plate-like form within the scope of the disclosure may be understood as a form having a dimension/extension in one direction, which is considerably lower than the dimension/extension in other directions. For example, the thickness of the body portion may be considerably lower than the size/extension in a longitudinal and/or transverse direction. The longitudinal direction within the scope of the disclosure may be understood as the length direction of the hand, that may be defined as a direction in a plane substantially parallel to the back of the hand in an extended state of the hand, connecting the middle of the wrist portion of the hand with the fingertip of the middle finger. The transverse direction within the scope of the disclosure may be understood as the direction orthogonal to the longitudinal direction in the plane substantially parallel to the back of the hand.

The thickness of the plate-like body portion of the sliding element may be in the range of about 0.4 mm to 2.5 mm, more specifically about 0.5 mm to 2.0 mm, further specifically about 0.6 mm to 1.5 mm. The size/ extension of the plate-like body portion in the longitudinal direction may be range of about 30 mm to 65 mm, more specifically about 35 mm to 60 mm, further specifically about 40 mm to 55 mm. The size/ extension of the plate-like body portion in the transverse direction may be range of about 6 mm to 25 mm, more specifically about 8 mm to 20 mm, further specifically about 10 mm to 15 mm. Other sizes are also possible. For example, in case of user wearable hand orthosis that is configured to be mounted on a hand of a child, the sliding element and specifically the plate-like body portion might have different length and/or width and/or height (thickness).

The thickness of the plate-like body portion of the sliding element may be substantially constant or may exhibit a variation in at least one direction. For example, the thickness of the plate-like body portion may increase or decrease toward the edges of the plate-like body portion in at least one direction. For example, the plate-like body portion may taper in the longitudinal and/or transversal direction toward the edges of the plate-like body portion.

Further, the platelike body portion may be substantially planar or may be curved. For example, the plate-like body portion may be slightly curved, to better conform to the shape of the back of the hand. Exemplary curvature may be in the range of about 60 mm to 300 mm, more specifically about 75 mm to 250 mm, further specifically about 90 mm to 150 mm. Alternatively and/or in addition, the plate-like body portion may be made of an elastic material and configured such that its curvature changes with applied pressure to match the curvature of the hand (which may itself change).

At least one major surface of the plate-like body portion, for example both of the major surfaces may be structured and/or may have elements and/or portions provided on it. For example, at least one major surface of the plate-like body portion may have at least one of at least one groove, at least one hole, projection or other structures. For example, at least one major surface of the plate-like body portion may be structured such as to form at least one of at least one finger module connection portion, at least one force transfer element connection portion and/or at least one guiding rail engagement portion. Alternatively or in addition, at least one of the at least one finger module connection portion, at least one of a force transfer element connection portion and at least one guiding rail engagement portion may be formed as separate elements that are connected by suitable means (for example by the use of adhesive) to the at least one major surface of the plate-like body portion of the sliding element.

Accordingly, the sliding element may further comprise at least one finger module connection portion, at least one of a force transfer element connection portion and/or at least one guiding rail engagement portion.

In an example, the support structure, for example at least the dorsal support portion and optionally the palmar support portion and/or wrist portion of the support structure may comprise at least one layer. The at least one layer may be a core layer (also referred to as a core skeleton layer). Alternatively or in addition, the at least one layer may be a low friction layer arranged on the side of the support structure facing the hand and/or on the side of the support structure opposite to side facing the hand. Additional layers, such as protective layers, reinforcement layers, cushioning layers, hardware layers, may also be provided.

The core layer may be configured to provide stability of the support structure and in particular to absorb compression forces exerted on the hand of the user. The core layer may be further configured to guide at least one force transfer element (such as a tendon) to the sliding element and/or at least one finger module and/or thumb module.

The core layer may have a core stiffness. Stiffness may be understood, within the scope of the disclosure unless individually differently specified, as the extent to which an object resists deformation in response to an applied force. The core stiffness may for example be an average stiffness of the core layer.

The core layer may be formed of at least one metal (e.g., low carbon steel), at least one elastomer, at least one polymer (e.g., silicone, thermoplastic urethane (TPU), thermoplastic elastomer (TPE), and/or PA-6 (polyamide 6)), and/or at least one non-textile material. However, it is understood that the core layer is not restricted to such exemplary materials. For example, the core layer may be formed from a first base material, such as for example 85A TPU, and a second base material, such as 95A TPU, having a greater stiffness than the first base material.

The core layer and/or the at least one additional layer may be a substantially uniform layer or may comprise a plurality of sections. For example, the core layer may be configured to form at least one of the portions and/or elements described herein. Non limiting examples include one or more of the following elements or portions: guiding rail, guiding groove, tendon guiding groove, guiding rail connection/fastening portion, support element connection/fastening portion, hyperextension prevention element, locking section, etc.

Alternatively or in addition, the core layer may be configured to form a plurality of section having different stiffness, flexibility, density, thicknesses, material compositions and/or or other properties. For example, the core layer and/or the at least one additional layer may be structured (e.g., by provision of holes, portions with different thicknesses such as groves, reinforcement ribs, projections, etc.), for example to form a plurality of zones with different mechanical properties, such as different stiffness, flexibility, etc. Thus, it may be possible to adapt the support structure very precisely to the needs of the user. Further it is possible to realize a lightweight and flexible, yet stable support structure.

For example, the core layer may comprise at least one reinforcement section stiffness greater than the stiffness of another section/part (such as a main section). The at least one reinforcement section may comprise at least one section having a larger thickness than the thickness of the at least one main section. The at least one reinforcement section may comprise more material and/or denser material than the at least one main section. The at least one reinforcement section may comprise material having a greater stiffness than material of the at least one main section. For example, one or more reinforcement sections may comprise at least one support element (such as reinforcement element) embedded into the core layer, such as for example spring steel (such as a low-carbon steel, low-alloy manganese or medium-carbon steel or high-carbon steel) insert element.

The core layer may comprise at least two reinforcement sections, wherein each of the at least two reinforcement sections may have a substantially identical stiffness. Substantially may be understood in the context of the present disclosure as including variations due to, for example, environmental and/or production factors. Alternatively, at least two reinforcement sections may have different stiffnesses from one another.

The core layer may further comprise at least one flexion section having at least a third stiffness smaller than for example the stiffness of a main layer section. The at least one flexion section may be a physical section of the core layer different from the at least one main layer section and/or the at least one reinforcement section. The at least one flexion section may comprise at least one section having a smaller thickness than the thickness of the at least one main layer section. The at least one flexion section may comprise less material and/or material of lower density than the at least one main layer section. For example, the at least one flexion section may comprise one or more through-holes through the core layer. The at least one flexion section may comprise material having a lower stiffness than material of the at least one main layer section. For example, one or more flexion sections may comprise a flexion element embedded into the core layer, such as for example a bending element and/or a hinge element.

The core layer may comprise at least two flexion sections, wherein each of the at least two flexion sections may have a substantially identical stiffness. Alternatively, at least two flexion sections may have different stiffnesses from one another.

The above description of various sections of the core layer applies also to at least one additional layer that may also exhibit a plurality of sections with different properties.

Alternatively or in addition to a core layer, the support structure and in particular the dorsal support portion, the palmar support portion and/or the wrist portion may comprise a low friction layer.

The low friction layer may for example be configured to reduce the friction between the movable sliding element and other parts of the support structure, such as for example the dorsal support portion and/or a cover portion. A low friction layer may also be configured to reduce the friction between parts of the support structure with a skin of the user's hand contacting these parts.

Accordingly, the low friction layer may be provided in parts of the support structure (e.g., in parts of the dorsal support portion and/or palmar support portion, and/or wrist portion) which come into contact with the hand and/or with the sliding element. Similarly, inner parts of the finger modules and/or the thumb module coming into contact with the hand of the user may be provided with a low friction layer.

The low friction layer may be for example a layer of Teflon^{®} PTFE, Nylon woven fabric, silk-blended fabrics, Lycra/spandex blended fabrics, etc.

Alternatively or in addition to a core layer and/or low friction layer, the support structure and in particular the dorsal support portion, the palmar support portion and/or the wrist portion may comprise other layers such as at least one protective layer, cushioning layer, hardware layer, reinforcement layer, etc.

In an example, at least the dorsal support portion and optionally the palmar support portion and/or wrist portion of the support structure may have a multilayer structure, i.e., may comprise at least two layers that may be configured to exhibit different properties and serve different purposes. For example, the multilayer structure may comprise at least a core layer as described above and at least one additional layer. The at least one additional layer may at least partially cover the core layer.

The at least one additional layer may be arrangeable and/or fixable adjacent to the core layer. At least one additional layer may be arrangeable and/or fixable directly adjacent to, preferentially in touching contact to, the core layer. Alternatively or in addition, at least one additional layer may be arrangeable and/or fixable indirectly adjacent to the core layer, wherein one or more intermediate elements and/or components may be arrangeable and/or fixable between the respective at least one additional layer and the core layer. Non-restricting examples of the one or more intermediate elements and/or components may comprise one or more other additional layers, one or more sensors, and/or one or more force transfer elements, etc.

The at least one additional layer may be configured to be detachable and/or removable from the core layer. In particular, the at least one inner additional layer may therefore be easily replaceable and/or cleanable. Therefore, such inner additional layers may improve hygienic properties and/or a lifetime of the user wearable hand orthosis.

Exemplary additional layers are low friction layer, protective layer, cushioning layer, hardware layer, reinforcement layer, etc. In case there are a plurality of additional layers, they may be formed of the same or different materials. The at least one additional layer may have properties (such as stiffness, flexibility, etc.) that are different from that of the core layer and/or different from that of other additional layers. For example, at least one additional layer may have a stiffness that is lower than that of the core layer. Further, one or more of the additional layers may be structured (e.g., by provision of holes, portions with different thicknesses such as groves or reinforcement ribs) in the same or different manner, for example to form a plurality of zones with different mechanical properties.

Thus, it may be possible to adapt the support structure very precisely to the needs of the user by selection of appropriate materials for the core layer and/or the at least one additional layer. For example, it may be possible to combine one or more materials of the core layer and/or the at least one additional layer into a three-dimensional composite structure to enable very precise control over the, e.g., mechanical, properties of the user wearable hand orthosis. In particular, it becomes possible to increase stiffness and/or rigidity in areas affected by high loads during operation of the user wearable hand orthosis, while maintaining softness against the user, e.g., a skin of the user, and flexibility in sensitive areas. In addition, this allows the user wearable hand orthosis to withstand significantly higher loads with the same degree of softness.

The at least one additional layer may be a low friction layer, such as the low friction layer described above. Further additional layers may also be provided between the core layer and the low friction layer or may be provided on the core layer.

The at least one additional layer may be an inner additional layer and there may be a plurality of inner additional layers. The at least one inner additional layer may be arrangeable at least partially on and/or fixable (e.g., directly or indirectly adjacent) to an inner, first side of the core layer. In particular, the inner side of the core layer may be a hand facing side of the core layer, preferentially in a use state of the user wearable hand orthosis. In other words, the at least one inner additional layer may be at least partially arrangeable between the core layer and the hand of the user, preferentially in the use state.

The at least one inner additional layer may comprise an inner fabric layer, wherein the inner fabric layer may be configured to prevent and/or reduce injury and/or discomfort of the user, such as for example caused by edges of the core layer during flexion of the hand. However, the inner fabric layer is not limited thereto. For example, the inner fabric layer may be configured to thermally insulate the hand of the user, e.g., to keep the hand of the user warm during low environmental temperatures, and/or the inner fabric layer may be configured to allow perspiration of the user to diffuse to the outside environment around the user (e.g., the inner fabric layer may be a high breathability fabric layer).

The at least one additional layer may comprise at least one outer additional layer. The at least one outer additional layer may be arrangeable at least partially on and/or fixable (e.g., directly or indirectly adjacent) to an outer, second side of the core layer. In particular, the outer side of the core skeleton layer may be a side of the core layer facing away from the user and/or the hand, preferentially in the use state. In other words, the at least one outer additional layer may be at least partially arrangeable such that the core layer is arrangeable and/or positionable between the hand of the user and the at least one outer additional layer, preferentially in the use state.

The at least one outer additional layer may comprise an outer fabric layer, wherein the outer fabric layer may be configured to prevent injury and/or discomfort of the user, such as for example caused by edges of the core layer during flexion of the hand. However, the outer fabric layer is not limited thereto. For example, the outer fabric layer may be configured to thermally insulate the hand of the user, e.g., to keep the hand of the user warm during low environmental temperatures, and/or to improve a breathability of the user wearable hand orthosis. Furthermore, the outer fabric layer may be configured to prevent and/or reduce damage to the user wearable hand orthosis and/or the core layer during use of the user wearable hand orthosis.

The at least one inner additional layer and/or the at least one outer additional layer may be user customizable. For example, a material choice of the at least one inner additional layer and/or the at least one outer additional layer may be made in light of the individual requirements of the user, such as in relation to allergies, skin conditions, and/or physiological properties of the user, e.g., sweating and/or temperature perception. Furthermore, optical and aesthetic properties of the at least one inner additional layer and/or the at least one outer additional layer may be chosen in light of user preferences and requirements, such as to facilitate mounting of the user wearable hand orthosis on the user.

While features and properties of the at least one additional layer are described and/or shown herein, it is understood that each further additional layer or each subsection of a particular additional layer may comprise any combination of features described and/or shown herein for the at least one additional layer.

The core layer and optionally at least one additional layer may be manufactured and/or manufacturable using molding or additive manufacturing. For example, the core layer may be manufactured, e.g., using one or more materials, by 3D printing the core layer. Specifically, the core layer and optionally at least one additional layer may therefore be easily and efficiently manufacturable. Furthermore, by providing a support structure having at least the core layer that is manufacturable by, e.g., 3D printing, the user wearable hand orthosis and/or the support structure can easily and locally be manufactured and/or adapted to the user, thereby increasing an adaptability and individualization of the user wearable hand orthosis.

Further, by allowing the core layer and optionally at least additional layer to be manufactured via, for example, 3D printing, the core layer may be precisely adapted to the specific requirements of the user, which may lead to a significant improvement of the fit of the user wearable hand orthosis. In addition, the core layer may easily be replaced without necessitating a replacement of the full user wearable hand orthosis.

The present disclosure is, however, not limited to manufacturing the core layer and optionally at least one additional layer using molding or additive manufacturing, but other suitable techniques such as injection molding, weaving, knitting, etc. may also be employed for the whole layers or at least one section of the respective layers.

The support structure may further comprise a cover portion connected or connectable to the dorsal support portion (and optionally to a wrist support portion), wherein the cover portion is movable between an opened and a closed position, wherein in the closed position a first surface of the cover portion arranged on the dorsal side (thus facing the second surface of the dorsal support portion) is configured to substantially cover the sliding element and optionally at least a connection portion of the at least one finger module when the at least one finger module is connected to the sliding element.

Specially, when in a closed position the cover portion may at least partially cover an upper surface (i.e., a surface on the side opposite to the dorsal side, i.e., on the side opposite to the side facing the hand) of the dorsal support portion. In an example, the cover portion covers substantially the whole upper surface of the dorsal support portion.

Thus, in a closed position of the cover portion, a surface of the cover portion facing the hand (the first surface of the cover portion) and the upper surface of the dorsal support portion (i.e., the second surface of the dorsal support portion) may form an enclosure (e.g., a pocket), which may enclose the sliding mechanism with the sliding element and optionally at least a connection portion of the at least one finger module when connected to the sliding element. In particular, when closed, the cover portion may create an ad-hoc Bowden tube for the sliding element. The sliding mechanism and in particular the sliding element and/or the connection portion of the at least one finger module can be thus protected when the cover portion is closed. Further, when closed the cover portion may constrain the sliding element and/or the at least one finger module. For example, when closed, the cover portion may restrict the movement of the sliding element to a movement in a plane substantially parallel to the plane of the hand back when the support structure is in a wear (use) state and/or may redirect the exerted force to the one or more finger modules connected to the sliding element.

The cover portion may be connected or may be connectable to an end (i.e., side) portion of the support structure (e.g., an end portion of the dorsal support portion and/or palmar support portion) in a transverse direction of the hand (and the support structure). The connection may be fixed or removable connection. To realize the connection between the cover portion and at least one other portion of the support structure, at least one hinge element configured to allow a rotational movement of the cover portion between an opened and a closed position may be provided.

The cover portion may have a multilayer structure comprising one or more of the layers described herein. For example, the cover portion may comprise a low friction layer arranged on the dorsal side (i.e., on the side of the cover facing the second surface of the dorsal support portion). Further, the multilayer structure of the cover may comprise additional layers, such as an inner layer, outer layer, core layer, etc. as described herein.

The low friction layer may at least partially cover the surface of the cover portion on the dorsal side of the cover portion. The low friction layer may be configured to reduce the friction between the movable sliding element and the cover. The low friction layer may be, for example, a Teflon^{®} PTFE layer, Nylon woven fabric, silk-blended fabrics, Lycra/spandex blended fabrics, etc. The low friction layer may be of the same material as those provided on the side of the dorsal support portion on which the sliding element is provided.

The user wearable hand orthosis may further comprise at least one support element, and/or a hyperextension prevention mechanism.

The at least one support element may be configured to bear compression load and may, for example exhibit stiffness that is higher than that of other components or sections of the support structure. The at least one support element may, for example, be a spring steel element, for example low-alloy manganese, medium-carbon steel or high-carbon steel insert element, element of carbon fiber, fiberglass, ABS/PETG, Nylon or other materials with similar stiffness properties. In an example, the at least one support element may have a substantially strip-like form, with thickness in the range of about 0.1 mm to 0.8 mm, more specifically about 0.2 mm to 0.6 mm, further specifically about 0.2 mm to 0.4 mm; width in the range of about 3 mm to 10 mm, more specifically about 4 mm to 8 mm, further specifically about 5 mm to 7 mm; and length in the range of about 30 mm to 80 mm, more specifically in the range of about 50 mm to 95 mm, further specifically in the range of about 60 mm to 80 mm.

The least one support element may be provided on or embedded in at least one of the dorsal support portion, palmar support portion and wrist portion. For example, the at least one support element may be provided on or embedded in the dorsal support portion. Specifically, the least one support element may be embedded in the core layer or may be connected to the core layer, as described above. The connection may be for example a fixed or a releasable connection. The at least one support element may also be an integral part of a reinforcement layer (as an example of an additional layer), which as described above may be connected to (fixedly or releasably) to a core layer.

In an example, the at least one support element may be provided between the core layer and the low friction layer and/or another additional layer (such as the above described inner or outer layer).

The at least one hyperextension prevention mechanism may be configured to limit or constrain the extend of movement of the sliding element in a direction toward the wrist of the hand, thus preventing hyperextension of the hand. The hyperextension prevention mechanism may consists of or may comprise at least one hyperextension prevention element arranged at an appropriate position, such as in the vicinity of the wrist of the user's hand, and configured to limit the movement of the sliding element in a direction toward the wrist, for example to prevent the movement of the sliding element beyond the first end position. The at least one hyperextension prevention element may, for example, be a mechanical stopper element.

The at least one hyperextension prevention element may be formed integrally with the dorsal support portion (e.g., may be formed of the same material as the dorsal support portion) or may be formed as a separate element fixedly or releasably connectable to the dorsal support portion. In an example, a connection point or portion of the guiding rail to the dorsal support portion may serve as a hyperextension prevention element. Thus, it is possible to realise a simple, yet efficient hyperextension prevention mechanism.

Similarly, the movement of the sliding element in a direction toward the fingers of the user's hand may be limited by a suitable stopper mechanism including at least one stopper element, such as for example at least one mechanical stopper element.

The at least one stopper element may be formed integrally with the dorsal support portion (e.g., may be formed of the same material as the dorsal support portion) or may be formed as a separate element fixedly or releasably connectable to the dorsal support portion. In an example, a connection point or portion of at least one guiding rail to the dorsal support portion and/or a connection point or portion of at least one support element may serve as a stopper element preventing the movement of the sliding element in a direction toward the fingers beyond the second end position. Thus, it is possible to realise a simple, yet efficient mechanism for limiting the movement of the sliding mechanism in a direction toward the fingers of the user's hand.

The support structure (e.g., the distal support portion and/or the palmar support portion and/or the wrist portion) and/or one or more of the finger modules and/or the thumb module may comprise one or more hardware mounting elements. The one or more hardware mounting elements may be configured to be engageable with one or more external hardware components. Non-limiting examples of external hardware components include one or more sensors and/or electronic equipment. The one or more sensors may be configured to sense a bending angle of one or more fingers of the hand, a pressure on the palm of the hand and fingers, and/or a motion of the hand. The electronic equipment may comprise a PCB, e.g., a flexible PCB, and/or a control unit, wherein the control unit may for example be configured to control the one or more sensors and/or the actuator. The control unit may be configured as a PCB, for example a flexible PCB. The one or more hardware mounting elements may for example comprise one or more sleeve elements, hook elements, loop elements, and/or clip-on elements.

In an example, the one or more hardware mounting elements may be mountable on or may be an integral part of the core layer.

In particular, by enabling such mounting of external hardware components directly on and/or in the core layer, a bulk and thickness of the user wearable hand orthosis may be reduced. Further, it may be possible to prevent or reduce sensation of the one or more external hardware components by the user and/or improve a mounting stability of the one or external hardware components.

For example, one or more external hardware components may be at least partially embedded into the core layer, preferentially fully embedded into the core layer. Thereby, a high structural stability of the user wearable hand orthosis may be achieved, and/or loss and/or unintentional removal of the one or more sensors and/or electronic equipment may be avoided.

It is also possible to mount or embed the one or more external hardware components in at least one dedicated layer, such as a hardware layer.

Another aspect of the invention relates to a method of manufacturing a user wearable hand orthosis comprising a support structure configured to be worn on a hand of a user. The user wearable hand orthosis may be the user wearable hand orthosis described herein and/or shown in the appended Figures.

Specifically, the method may include providing a support structure, a sliding mechanism comprising a sliding element and at least one finger module configured to be connectable or be connected to the sliding element, as described herein in connection with the first aspect and its examples and embodiments and/or shown in the appended Figures.

Specifically, the method may include:
providing a support structure comprising a dorsal support portion configured to be worn on at least a part of a back of the hand of the user, wherein the dorsal support portion has a first surface on a dorsal side of the dorsal support portion (i.e., the side facing the back of the hand) and a second surface on a side of the dorsal support portion opposite to the dorsal side (i.e., is opposite to the first surface);
providing a sliding mechanism comprising a sliding element configured to move (slide) along a track on the second surface of the dorsal support portion between a first end position and a second end position, wherein in a use state of the user wearable hand orthosis the first end position is arranged proximal to a wrist portion of the hand of the user and the second end position is arranged at a distance from the first end position in a direction toward a knuckle joint portion of the hand of the user;
providing at least one finger module configured to be worn on at least one finger of the hand of the user, wherein the at least one finger module comprises a distal end portion configured to be worn on at least one distal section of the at least one finger and a sliding element connection portion configured to be connectable or be connected to the sliding element.

The above steps may be executed in a different order, in particular, in an arbitrary order.

The method may in particular comprise any combination of features of the user wearable hand orthosis and/or may exhibit the advantages of the user wearable hand orthosis, as described herein and/or shown in the appended Figures.

The providing of the support structure and/or sliding mechanism and/or at least one finger module may include selecting and/or retrieving a premanufactured support structure and/or sliding mechanism and/or at least one finger module from a storage. Alternatively or in addition, the providing of a support structure and/or sliding mechanism and/or at least one finger module may include manufacturing of the support structure and/or the sliding mechanism and/or the at least one finger module, for example by using known methods, such as additive methods, 3D printing, moulding (such as injection moulding), knitting, etc. The providing of a support structure may comprise connecting the individual portions of the support structure to each other.

The method may further comprise connecting the sliding mechanism to the dorsal support portion, wherein the connecting of the sliding mechanism to the dorsal support portion may optionally include movably mounting the sliding element on a guiding rail arranged on a surface of the dorsal support portion that is opposite to a dorsal surface of the dorsal support portion.

The method may also comprise connecting at least one sliding element connection portion of the at least one the finger module to the sliding element, in particular to a connection portion of the sliding element.

The step of connecting of the at least one sliding element connection portion of the at least one the finger module to the sliding element may comprise adjusting the length from a connection portion (anchor point or portion) of the at least one finger module to the distal end portion of the at least one finger module.

The length may be adjusted by for example changing the pairing(s) between connection element(s) provided on the at least finger module and connection element(s) provided on the sliding element. The connection elements may, for example, include holes (e.g., provided in the at least one finger module) and the corresponding grips (e.g., provided on the sliding element) and the pairing between the holes and the grips may be changed, in order to change or adjust the length from a connection portion (anchor point or portion) of the at least one finger module to the distal end portion of the at least one finger module. The connection may also be realized by at least one adjustable clamp, in which case the length adjustment may be performed by a technician during the initial fitting of the product, for example.

Thus, the user wearable hand orthosis may be easily adapted to the needs and/or anatomy of the individual user.

The method may also comprise connecting the sliding element to at least one force transfer element configured to exert a (pulling) force on the sliding element. As described herein, the connection may be a fixed or releasable connection and may be realized by appropriate connection elements and/or portions.

Providing the support structure may comprise providing a multilayer structure such as the one disclosed in connection with the user wearable hand orthosis. In particular, providing the support structure may comprise:
providing a core layer; and/or
providing at least one additional layer, wherein the at least one additional layer may be at least one low friction layer arranged on the dorsal side and/or on the side opposite to the dorsal side of the core layer.

The providing of a core layer may comprise the providing of a core layer of a dorsal support portion and/or palmar support portion and/or wrist support portion.

The providing of a core layer may include manufacturing of the core layer using molding and/or additive manufacturing (such as 3D printing) and/or other manufacturing methods such as knitting, weaving, etc. Thereby an easy and adaptable manufacture of the core layer may be achieved. Furthermore, by using, for example, additive manufacturing, such as 3D printing, the core layer can be locally and rapidly be produced. The step of providing at least one additional layer may include manufacturing the at least one additional layer, for example using one or more of the manufacturing methods mentioned in connection with the manufacturing of the core layer. The steps of manufacturing the core layer and manufacturing of at least one additional layer may be undertaken at least partially substantially simultaneously and/or concurrently.

The providing of the core layer and/or the at least one low friction layer and/or additional layer may comprise coating or permanently fixing the at least one low friction layer and/or additional layer on at least a portion of at least one surface (e.g., surface on the dorsal side and/or surface on the side oppositive to the dorsal side) of one of the layers of the multilayer structure such as a core layer.

The providing of the core layer and the at least one low friction layer and/or additional layer may comprise connecting (e.g., by suitable fastening or locking elements or portions) the at least one low friction layer and/or additional layer to at least a portion at least one surface (e.g., surface on the dorsal side and/or surface on the side oppositive to the dorsal side) of core layer.

The step of manufacturing the core layer may further comprise providing at least one of the portions and elements described herein on and/or in the core layer, such as for example force transfer element guide element (e.g., tendon guide element),

The step of manufacturing the core layer may further comprise forming a plurality of sections, such as the sections described herein in connection with the use wearable hand orthosis (e.g., finger module connection portion, force transfer element connection portion, guiding element connection portion, support element connection portion, force transfer element guide element, etc.). Alternatively or in addition, the step of manufacturing the core layer may comprise forming at least one main layer section having a first stiffness and at least one reinforcement section having at least a second stiffness greater than the first stiffness. The step of manufacturing the core layer may further comprise forming at least one flexion section having at least a third stiffness smaller than the first stiffness. Thus, it is possible to better adapt the user wearable hand orthosis and in particular the support structure to the need of the user.

The method may further comprise wrapping the support structure substantially around the hand to bring a first locking section of the support structure into engagement with a second locking section of the support structure to mount the support structure on the hand of the user. Thereby, a simple and efficient mounting of the user wearable hand orthosis on the user may be achieved.

Wrapping substantially around the hand of the user may be understood such that at least part of the support structure may be deformed around the hand of the user, preferentially to at least partially conform to a shape of the hand. The first locking section and the second locking section may be provided for example on the dorsal support portion and the palmar support portion, respectively.

As described above, the first locking section and the second locking section may comprise at least one hook and at least one loop configured to be fixable to one another to bring the first and second locking sections into engagement with one another. However, the first and second locking sections are not limited thereto, and other types of locking mechanisms may be implemented, such as for example Velcro-based locking mechanisms, clamps, etc.

The invention will now be further explained using exemplary embodiments illustrated in the appended Figures. However, the embodiments shown in the appended Figures and/or described below are to be understood as exemplary only, and the invention is therefore not to be interpreted as limited to such exemplary embodiments.

The Figures show:
- **Figure 1:**: a schematic top view of an exemplary sliding element;
- **Figure 2A:**: a schematic isometric view showing the top surface of an exemplary sliding element according to Figure 1;
- **Figure 2B:**: a schematic isometric view showing the top surface of an exemplary sliding element according to Figure 1 in a state where a tendon is connected;
- **Figure 3A:**: a schematic isometric view showing the bottom surface of an exemplary sliding element according to Figure 1;
- **Figure 3B:**: a cross-sectional view of an exemplary sliding element according to Figure 1;
- **Figure 4:**: a schematic view of an exemplary support structure, wherein the sliding element is in a first position;
- **Figure 5:**: a schematic view of an exemplary support structure according to Figure 4, wherein the sliding element is in a second position;
- **Figure 6:**: a schematic perspective view of an exemplary support structure according to Figure 5 with connected finger modules;
- **Figure 7:**: a schematic top view of an exemplary support structure according to Figure 5;
- **Figure 8**: a schematic top view of components of an exemplary support structure prior to mounting a sliding mechanism;
- **Figure 9**: a schematic top view of components of an exemplary support structure according to Figure 8, wherein the sliding mechanism is being mounted on the support structure;
- **Figure 10A**: a schematic view of an exemplary user wearable hand orthosis in a use state with a cover closed;
- **Figure 10B**: a schematic view of an exemplary user wearable hand orthosis in a use state with a cover opened; and
- **Figure 11:**: an exemplary flow chart of a method of manufacturing a user wearable hand orthosis.

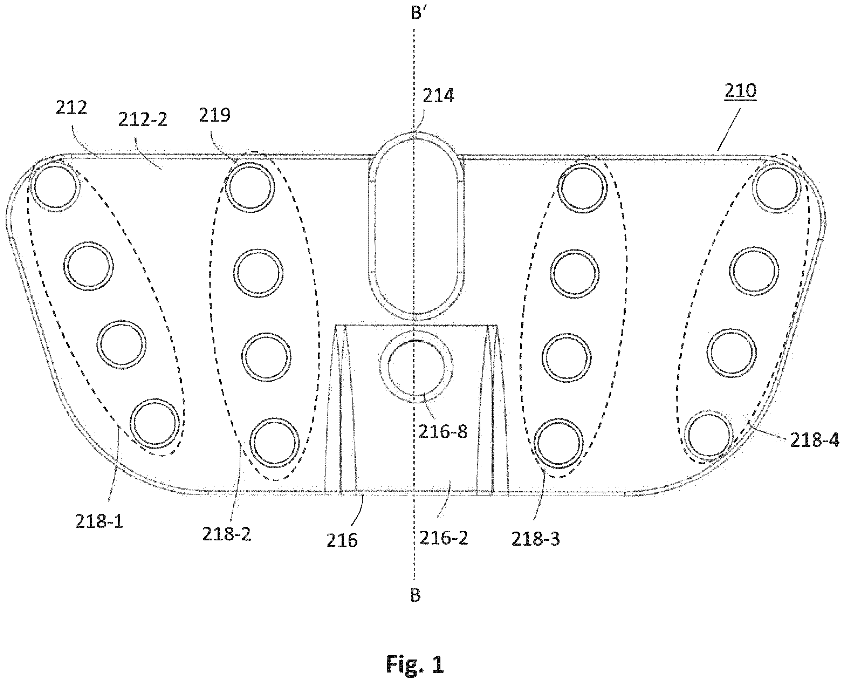

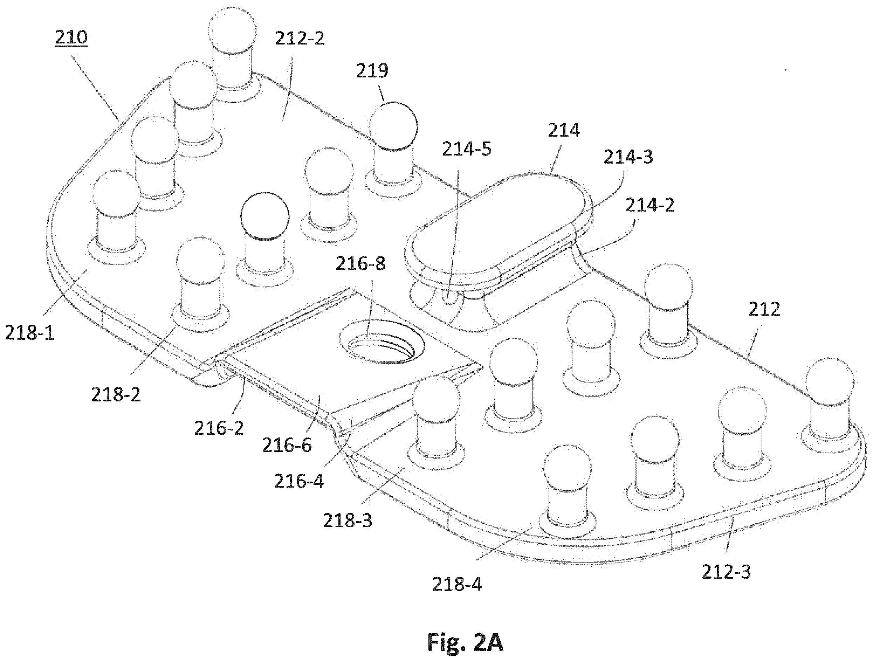

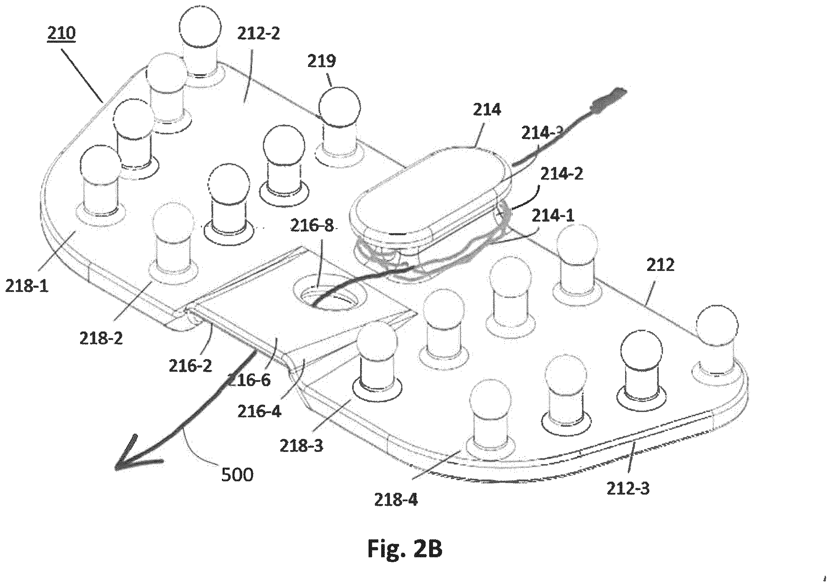

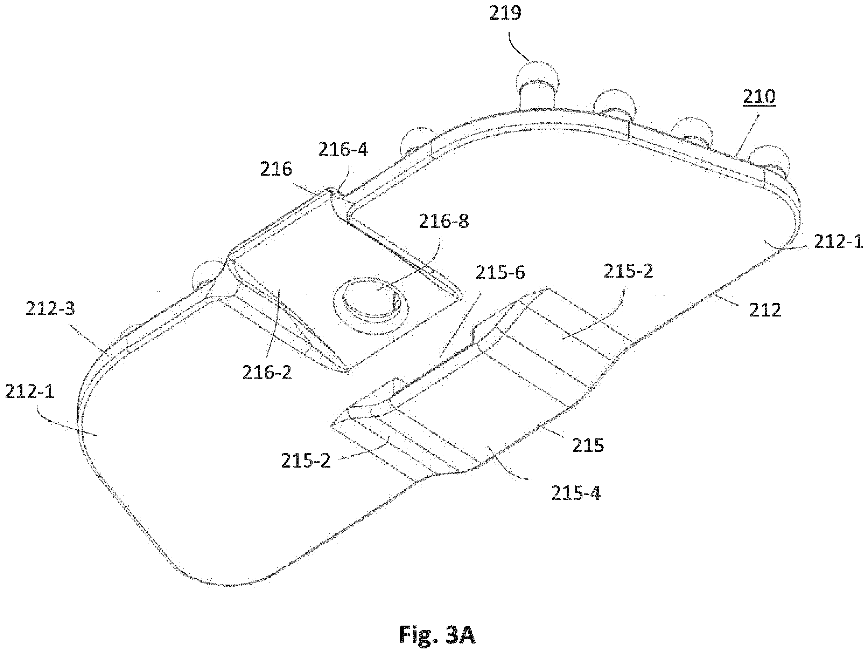

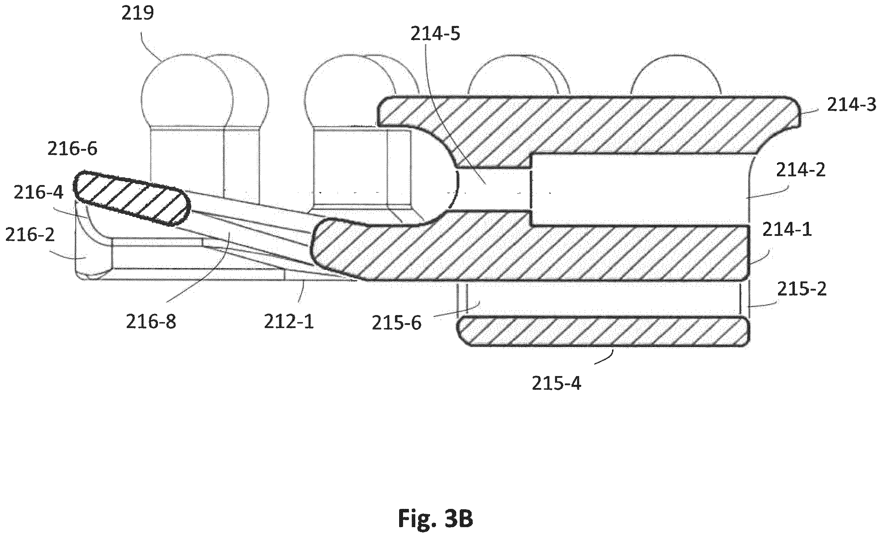

**Figures 1** to **3** show schematic views of an exemplary sliding element 210 of an exemplary sliding mechanism. **Figure 1** shows a schematic top view of the exemplary sliding element 210, i.e., a schematic view of the side (top side) of the sliding element 210 that is opposite to the side facing the hand back of the hand in use state of the user wearable hand orthosis, i.e., opposite to the dorsal side. **Figure 2A** shows a schematic isometric top view of the sliding element 210 shown in Figure 1, i.e., a schematic isometric view of the top side of the sliding element 210 . **Figure 2B** shows a schematic isometric top view of the sliding element 210 shown in Figure 1 in a state where a tendon is connected. **Figure 3A** shows a schematic isometric view showing the bottom side of the exemplary sliding element 210, i.e., the dorsal side of the sliding element 210, i.e., the dorsal side of the sliding element 210. **Figure 3B** shows a cross-sectional view of an exemplary sliding element according to Figure 1 along the line B-B' running through the middle of the sliding element 210 in a plane substantially orthogonal to the major surfaces of the sliding element 210.

The sliding mechanism is comprised by an exemplary user wearable hand orthosis (not shown in the Figures). The user wearable hand orthosis shown in the Figures is an exemplary tendon-driven glove orthosis configured to be worn on a hand of a user. In this exemplary tendon driven glove orthosis the at least one force transfer element is a tendon. However, other types of user wearable hand orthosis (such as rigid user wearable hand orthosis) and/or other force transfer elements are also possible. The at least one force transfer element (not shown in the figures) may be a part of the user wearable hand orthosis.

The sliding element 210 comprises a body portion 212 having a substantially plate-like form. Substantially plate-like form as understood within the scope of the disclosure relates to a form having a dimension/extension in one direction, which is considerably lower than the dimension/extension in other direction. In particular, the thickness of the body portion 212 may be considerably lower than the size/extension in a longitudinal direction L and/or transverse direction T. The longitudinal direction L as understood within the scope of the disclosure relates to a length direction of the hand, that may be defined as a direction in the plane substantially parallel to the back of the hand in an extended state of the hand, which connects the middle of the wrist portion of the hand with the fingertip of the middle finger. The transverse direction T as understood within the scope of the disclosure relates to a direction orthogonal to the longitudinal direction L in the plane substantially parallel to the back of the hand.

The body portion 212 has major surfaces, a first major surface 212-1 and a second major surface 212-2. The major surfaces 212-1 and 212-2 may be substantially planar surfaces and may be substantially parallel to each other. The first major surface 212-1 is on dorsal side of the sliding element 212, i.e, on the side of the sliding element 212 facing the back of the hand in a use state of the user wearable hand orthosis. The second major surface 21-2 is on the side opposite to the dorsal side, i.e., on the side facing away of the back of the hand in a use state of the user wearable hand orthosis. The body portion 212 has further a peripheral (side) surface 212-3, which connects the first major surface 212-1 and the second major surface 212-2. In the example shown in the Figures, the body portion 212 has a substantially trapezoidal shape with rounded corners. Other shapes, such as for example rectangular, quadratic, round, oval, etc. shapes with or without rounded edges are also possible.

The thickness (height) of the body portion 212 may be in the range of about 0.4 mm to 2.5 mm, more specifically about 0.5 mm to 2.0 mm, further specifically about 0.6 mm to 1.5 mm. The (maximal) size/ extension of the body portion 212 in the longitudinal direction L may be range of about 30 mm to 65 mm, more specifically about 35 mm to 60 mm, further specifically about 40 mm to 55 mm. The (maximal) size/ extension of the body portion in the transverse direction T may be range of about 6 mm to 25 mm, more specifically about 8 mm to 20 mm, further specifically about 10 mm to 15 mm.

The sliding element 210 further comprises a tendon connection portion 214 (as an example of a force transfer element connection portion 214), a guiding rail engagement portion 216 and a plurality of finger module connection portions 218-x (x = 1, ...4).

The tendon connection portion 214 is configured such that one or more tendons 500 (as an example of force transfer element(s)) are mountable and may be fixed to or on the tendon connection portion 214.

In the example shown in the Figures, the tendon connection portion 214 is realized as a hitch with an anchor point for the tendon running through the center.

The tendon connection portion 214 has an elongated "mushroom" like form comprising a base portion 214-1, an intermediate (stem) portion 214-2 and a head portion 214-3. The edges of the tendon connection portion 214 may be rounded, e.g., to prevent injury and/or damage of other parts of the user wearable hand orthosis.

The base portion 214-1 of the tendon connection portion extends from and is connected to (fixedly or releasably) the second major surface 212-2 of the body portion 212. The intermediate (stem) portion 214-2 extends from the base portion 214-1 and connects the base portion 214-1 with the head portion 214-3. The cross-section of the intermediate portion 214-2 of the tendon connection portion 214 in a plane substantially parallel to the plane of the second major surface 212-2 has a substantially elliptical or oval form, wherein deviations from this form and other forms are also possible. The intermediate portion 214-2 is provided with a through hole 214-5 for fixing the tendon 500 in a peripheral surface thereof.

The head portion 214-3 of the tendon connection portion 214 is connected to and extends from the intermediate portion 214-2 in a direction away from the second major surface 212-2 of the body portion 212. In a plane substantially parallel to the second major surface 212-2 of the body portion, a peripheral surface of the head portion 214-3 extends over, i.e., beyond the peripheral surface of the intermediate portion 214-2. In other words, the head portion 214-3 exhibits greater diameter(s) than the intermediate portion 214-2. Thus, the tendon may be held securely in place when connected.

The end portion of a tendon 500 may be securely fixed to the tendon connection portion 214. For example, as shown in Figure 2B, the end portion of the tendon 500 may be wrapped around the intermediate portion 214-2 of the tendon connection portion 214 and securely fixed to the tendon connection portion 214. Thus, it may be possible to adjust the length of the tendon, for example depending on the needs of the individual user.

In particular, the tendon connection portion 214 may be realized as a hitch with an anchor point for the tendon running through the center. The end of the tendon 500 may be crimped with a small steel alloy tube (for example: 0.6 mm ID x 1.0 mm OD x 3mm length) which is inserted through a through hole 214-5 in the center of the hitch which the crimp cannot pass through. The slack tendon 500 may be then wound around the hitch 2-3 times to take direct stresses off of the crimp anchor, as shown in Figure 2B The tendon 500 may be passed over these loops before being fed through the opening 216-8 in a top plate of a motion extension portion 216-6, thereby preventing the loops from coming undone.

The tendon connection portion 214 is not restricted to the above form and may have different forms and/or sizes. Further, other types of tendon connection portions may be employed.

The sliding element 210 may further comprise a guiding rail engagement portion 215 configured to engage with at least one guiding rail 220 of the sliding mechanism.

In the example shown in the Figures, the guiding rail engagement portion 215 comprises a pair of side walls 215-2 projecting from the first major surface 212-1 in a in a direction toward a surface of the dorsal portion 110 on which the sliding mechanism with the sliding element 210 is mounted. The side walls 216-2 of the guiding rail engagement portion 215 may extend at an angle from the first major surface 212-1 of the body portion 212 equal to or different from 90 degrees, such as an angle smaller than 90 degrees. The side walls 215-2 are connected by a bottom plate 215-4. In the example shown in the Figures the bottom plate 215-4 has a substantially rectangular form. The side walls 215-2 and the bottom plate 215-4 of the guiding rail engagement portion 215 enclose a guiding rail accommodation space 215-6 that is open on two opposite ends thereof, in order to allow a passage of the guiding rail 220. The guiding rail accommodation space 215-6 is configured such that the guiding rail 220 is insertable in the guiding rail accommodation space 215-6, thereby engaging the sliding element 210 with the guiding rail 220 in such a way as to enable a sliding movement of the sliding element 210 along the guiding rail 220. The first major surface 212-1 of the sliding element 210 and the bottom plate 215-4 restrict an out-of-plane movement of the sliding element 210. The side walls 215-2 restrict the movement of the sliding element 210 in a transverse direction.

The sliding element 210 may optionally further comprise a motion extension portion 216 configured to allow the sliding element 210 to be pulled farther back toward the wrist of the hand without being forced into out-of-plane motion. Further, by the provision of a motion extension portion 216, it is possible to realize a sliding element 210 with sufficient size and therefore stability, while having a sufficient motion range.

In the example shown in the Figures, the motion extension portion 216 comprises an opening 216-2, which is formed by a through opening in the first major surface 212-1, in the second major surface 212-2 and in the peripheral surface 212-3 of the body portion 212. Further, the motion extension portion 216 comprises side walls 216-4 surrounding the opening 216-2 and connected to the second major surface 212-2 of the body portion 212, wherein the side walls 216-4 extend in a direction away from the dorsal side of the body portion 212. The side walls 216-4 of the motion extension portion 216 may extend at an angle from the second major surface 212-2 of the body portion equal to or different from 90 degrees, such as an angle smaller than 90 degrees.

The side walls of the opening 216-2 formed in the body portion and/or the side walls 216-2 surrounding the opening form together a peripheral wall portion of the motion extension portion 216. The peripheral wall portion may optionally be provided with at least one projection and/or groove or recess that engages with a corresponding groove or projection on the guiding rail 220.

Further, the motion extension portion 216 comprises a top plate 216-6 having a substantially rectangular form. The top plate 216-6 is connected on two opposite sides thereof to the respective one of the side walls 216-4. On a third side thereof, the top plate 216-6 is connected to the second major surface 212-2 of the body portion 212. The top plate 216-6 extends from the second major surface 212-2 at an angle different from 90 degree (i.e., the top plate 216-6 is slanted with respect to the second major surface 212-2). A fourth side of the top plate 216-6 (opposite to the third side) is substantially free (not connected to another part or element) and extends over the opening 212-2.

In the example shown in the Figures the height of the side walls side walls 216-4 of the motion extension portion 216 reduces gradually from a first end portion arranged in flush or in proximity to the peripheral surface 212-3 of the body portion 212 in a direction toward a connection part between the top plate 216-6 of the motion extension portion 216 and the second surface 212-2 of the body portion 212.

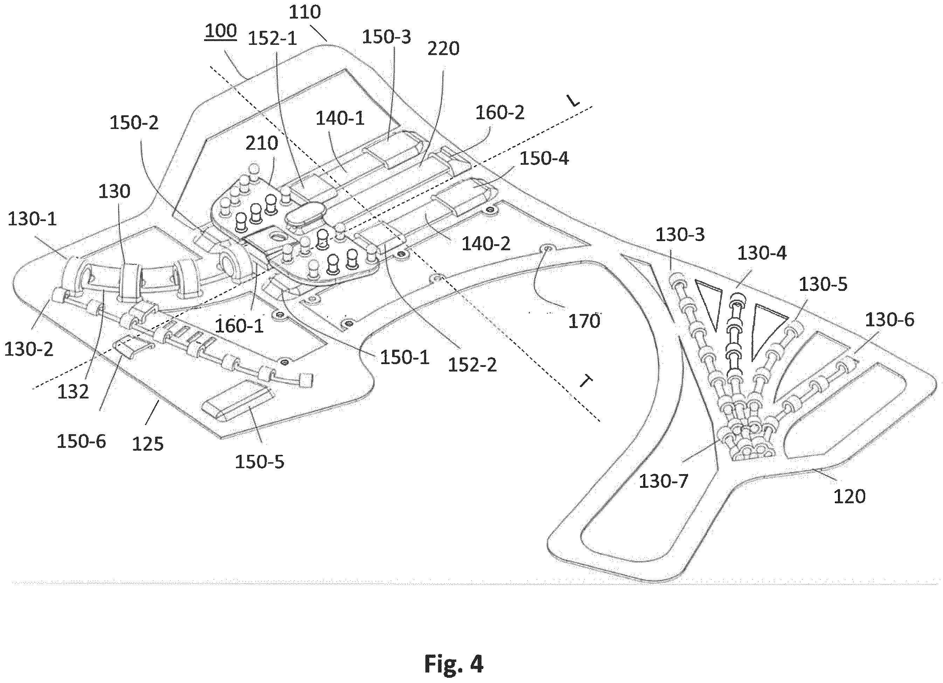

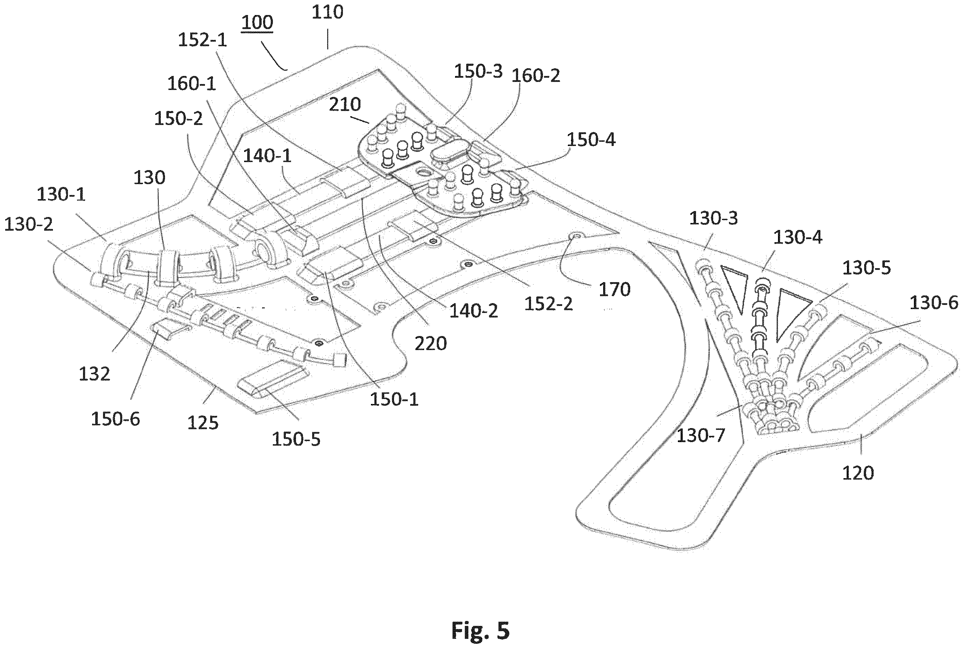

The peripheral wall portion and the top plate 216-6 of the motion extension portion 216 surround an accommodation space that is such as to at least partially accommodate a stopper element, thereby allowing the sliding element 210 to at least partially slide over the stopper element. The stopper element may for example be a mechanical stopper element 160-1 shown in Figures 4 and 5 or any other backstop element configured to limit the motion of the sliding element 210 toward the wrist of the hand. Further, the accommodation space may be configured such as to allow the passage of the at least one tendon 500, so that the at least tendon 500 does not obstruct the engagement of the sliding element 210 with the at least one guiding rail 220 and the movement of the sliding element 210 along the guiding rail 220. To allow the passage of the at least one tendon, the top plate 216-6 may be provided with a through opening 216-8. Thus, for example, a more direct route to a Bowden tube in the glove may be realized.

The edges of the motion extension portion 216 may be rounded, e.g., to prevent injury and/or damage of other parts of the user wearable hand orthosis.

In the example shown in the Figures, the motion extension portion 216, the guiding rail engagement portion 215 and the tendon connection portion 214 are arranged in a middle section of the body portion 212 in the transverse direction T. The motion extension portion 216 is arranged on the side of the sliding element 210, which is closer to the wrist of the hand of the user in use state of the user wearable hand orthosis. The tendon connection portion 214 and the guiding rail connection portion 215 are arranged on the side of the sliding element 210 that is closer to the knuckle joints of the hand of the user in use state of the user wearable hand orthosis. In other words, the motion extension portion 216 is closer to a wrist portion of support structure than the tendon connection portion 214 and/or the guiding rail engagement portion 215. Other arrangements of the motion extension portion 216, guiding rail engagement portion 215 and/or tendon connection portion 214 are also possible.

The sliding element 210 further comprises at least one finger module connection portion 218-x (x = 1, ...4). Each finger module connection portion 218-x is configured to be engageable with one or more corresponding connection portions 340-x of at least one finger module 300 to fix the at least one finger module 300 to the sliding element 210.

In the example shown in the Figures, the sliding element comprises a plurality of finger module connection portions 218-x (specifically four finger module connection portions 218-1 to 218-4), each finger module connection portion 218-x (x = 1,....4) configured for connection of different one of the plurality of finger modules 300 to the sliding element 210. It is, however, possible to have different number of finger module connection portions, for example one finger module connection portion.

In the example shown in the Figures, each finger module connection portion 218-x comprises a plurality of finger module connection elements 219. It is, however, possible to realize finger module connection portions having one finger module connection element 219. The finger module connection elements 219 in each finger module connection portion 218-x may be arranged in a row in the longitudinal direction L. A respective finger module 300 may be connected by engaging one of the finger module connection elements 219 of the respective finger module connection portion 218-x to a corresponding connection element 342 of a sliding element connection portion 340 of the finger module 300. By changing the pairings between a finger module connection element 219 of the sliding element and a respective element (sliding module connection element 342) of the finger module, it is possible to adjust or vary the length between the connection point or portion of the respective finger module 300 to the tip of the finger module, e.g., to accommodate for different hand anatomies and/or different needs for hand extensions of the individual users. Accordingly, it is possible to customize the hand orthosis in a simple and efficient manner.

In the example shown in the figures there are four finger module connection portions 218.x, each of them having four finger module connection elements 219. Two of the finger module connection portions (finger module connection portions 218-1 and 218-2) are arranged on one side of the guiding rail engagement portion 116 and the tendon connection portion 114 and two of the finger module connection portions (finger module connection portions 218-3 and 218-4) are arranged on the other side of the guiding rail engagement portion 116 and the tendon connection portion 114. However, the present description is not limited to this example and different number of finger module connection portions and/or different numbers of finger module connection elements in each finger module connection portion may be provided. The arrangement of the finger module connection portions and/or the finger module connection elements may also differ.

The finger module connection elements 219 may be any suitable connection elements. For example, each finger module connection element 219 may be configured as a peg with a rounded head that is engageable with one or more peg-receiving recess or opening 342 of the at least one finger module 300. Such an exemplary configuration may allow a reliable transfer of forces and/or a high resistance of connection quality and safety to degradation over time.

However, the at least one finger module connection element 219 is not limited to the exemplary embodiment shown in the Figures. For example, the finger module connection element 219 may be configured as a peg-receiving recess or opening and which is engageable with a corresponding peg provide in the connection portion of the respective finger module 300. Alternatively or in addition, other types of connection elements 219 of the sliding element and corresponding elements 342 of the finger module 300 may be employed, such as for example connection elements having different form, dimensions, materials and/or connection elements of different type, such as for example clamping elements, Velcro elements, etc.

The sliding element is not limited to the sliding element described above, but further modifications are also possible. Non-limiting examples of such modifications include one or more of the following modifications:
In the above example, the sliding mechanism comprises one sliding element 210 which is movable along a single guiding rail 220. The sliding element 210 may also be configured to be movably mountable on a plurality of guiding rails, for example on two guiding rails. This may improve the stability of the sliding mechanism. The sliding mechanism may also comprise a plurality of (i.e., two or more) sliding elements 210 that are movable along respective one or more guiding rails or along one or more other guiding elements such as guiding grooves or recesses. It is also possible to omit the guiding rails (or any other guiding elements such as guiding grooves, recesses, etc.) altogether.

In the above examples, the user wearable hand orthosis may comprise a single force transfer element (e.g., a tendon) connected or connectable to the sliding element 210, thus realizing one-to-many connection (via the sliding element) between an actuator and the at least one finger module 300 connected to the sliding element 210. It is, however, also possible to employ a plurality of (i.e., two or more) force transfer elements (e.g., tendons) connected to or connectable to the sliding element 210.

Further, the shape, geometrical dimensions, materials and/or other properties of the sliding element 210 and its components (such as the body portion 212, the force transfer element connection portion 214, guiding rail engagement portion 216, finger module connection portions, etc.) are not restricted to the ones described.

For example, the thickness plate-like body portion 212 of the sliding element 210 need not be substantially constant in a longitudinal and/or transverse direction (except for the places where openings and/or protrusions are provided). The thickness of the plate-like body portion may vary in a longitudinal and/or transverse direction. For example, the plate-like body portion 212 may taper in the longitudinal and/or transversal direction toward its edges.

Further, the major surfaces 212-1 and 212-2 of the plate-like body portion 212 need not be substantially plane surfaces, but may be curved, to better conform to the shape of the back of the hand. Alternatively or in addition, the plate-like body portion 212 may be made of a flexible material, in order to allow the curvature of the body portion 212 to change with applied pressure to match the curvature of the hand, which itself may change.

In the above examples, the body portion 212, the tendon connection portion 214, the guiding rail engagement portion 216 and the finger module connection portions 218 are formed integrally and may be from the same material. At least one of the tendon connection portion 214, the guiding rail engagement portion 216, the finger module connection portions 218 may also formed as a separate element/separate elements that is/are connected to at least one major surface of the body portion 212, for example by the use of adhesive or other fastening means. In this case, at least one of the tendon connection portion 214, the guiding rail engagement portion 216, the finger module connection portions 218 may be formed from a different material than the body portion 212. Each of the above portion may itself be formed of different materials.

The sliding element 210 is movably mounted on or connected to a support structure 100 and more specifically to a dorsal support portion 110 of the support structure 100.

**Figures 4** and **5** show a schematic top view of an exemplary support structure 100 with a sliding mechanism mounted on it, i.e., a schematic view of the side of the support structure 100 that is opposite to the side facing the hand back in the use state of the user wearable hand orthosis, i.e., opposite to the dorsal side. The dorsal side of the support structure may be made substantially smooth and/or may optionally be covered with at least one additional layer, such as a padding layer, an outer layer, a low friction layer, etc., as described herein.

The sliding mechanism comprises a movable sliding element 210, such as for example described in connection with Figures 1 to 3. Figure 4 shows a schematic top view of the support structure 100 with the sliding mechanism, wherein the sliding element 210 is in a first end position and Figure 5 shows a schematic view of the support structure with the sliding mechanism, wherein the sliding element 210 is in a second end position. The sliding element 210 is movably mounted on a guiding rail 220.

Figures 4 and 5 show the support structure 100 as not yet being mounted on the hand of the user. When not in use, the support structure 100 shown is substantially planar. The support structure 100 is arrangeable on the hand and optionally the wrist of the user. For example, the support structure may be wrappable around the hand and optionally the wrist of the user. This may, on one hand, allow easy and safe storage of the support structure when not in use, and, on the other hand, simplify designing and/or manufacturing of the support structure (e.g., additional hardware may be more easily mountable on a planar support structure).

The support structure 100 comprises a dorsal support portion 110, a palmar support portion 120 and a wrist portion 125. The dorsal support portion 110 is configured to be mounted on the back of the user's hand. The palmar support portion 120 is configured to be mounted on the palm of the user's hand. The wrist portion 125 is configured to be mounted on the wrist of the user's hand (e.g., be at least partially wrapped around the wrist). The dorsal support portion 110, the palmar support portion 120 and the wrist portion 125 may for example be of the same material and may be formed as one integral structure. The dorsal support portion 110 and/or the palmar support portion 120 and/or the wrist portion 125 may be formed as separate modules that are connectable to each other (e.g., releasably connectable). The connection of the dorsal support portion 110 and/or the palmar support portion 120 and/or the wrist portion 125 may be realized by any known means, for example by Velcro straps, pegs and corresponding peg receiving recesses, clamps, etc.

The dorsal support portion 110 and/or the palmar support portion 120 and/or the wrist portion 125 may have a plurality of sections with different properties, such as sections having different stiffness, flexibility, etc. For example, the dorsal support portion 110 and/or the palmar support portion 120 may have at least one cut-out or opening and/or at least one section with increased flexibility arranged and configured such as to improve the mountability and/or wearability of the support structure, while assuring sufficient stability.

One or more of the dorsal support portion 110, the palmar support portion 120 and the wrist portion 125 may comprise at least one tendon guide element 130 (exemplary force transfer element guide element). Each tendon guide element 130 may be configured to guide at least one tendon along a surface of the dorsal support portion 110 and/or the palmar support portion 120 and/or wrist portion 125, in particular along the surface that is on the side of the support structure opposite to the dorsal side. Each tendon guide element 130 may have one central cavity or opening to guide a tendon therein or may have a plurality of central cavities or openings to guide a plurality of tendons therein. One or more of the tendon guide elements 130 may define a corresponding tendon routing path for a respective tendon. Furthermore, the surface on which the at least one tendon guide element 130 is arranged may further comprise at least one tendon groove 132. Each tendon groove 130 may extend at least partially along a tendon routing path and may further be configured to accommodate a respective tendon at least partially along its corresponding tendon routing path.

In the example shown in Figures 4 and 5, each of the wrist portion 125 and the palmar support portion 120 comprises a plurality of tendon guide elements. The plurality of tendon guide elements 130 are arranged in groups, wherein each group forms a routing path for one tendon. For example, the wrist portion 125 comprises a first group of tendon guide elements 130-1 configured to form a routing path for at least one tendon that is connectable to the sliding element 210 and more specifically to the tendon connection portion 214.

The wrist portion 125 may further comprise a second group of tendon elements 130-2 configured to form a routing path for at least one additional tendon that is connectable to a thumb module. The palmar support portion may comprise four groups of tendon elements 130-3 to 130-6, each of them configured to form a routing path for at least one additional tendon that is connectable to each of the finger modules. The palmar support portion may also comprise an additional group 130-7 of tendon elements configured to form a routing path for at least one additional tendon that is connectable to a thumb module. The additional tendons are examples of additional force transfer elements described elsewhere.

It is noted that the present disclosure is not limited to the above tendon routing paths. Instead, the number of tendon routing paths may be determined on the basis of the number of required and/or desired tendons. Therefore, more or less than the described tendon routing paths may be implemented. Further, also the dorsal support portion 110 may be provided with one or more tendon guide elements that may be arranged in one or more groups as described above.

The at least one tendon element guide element 130 may be made from the same material as a core layer (described in further detail elsewhere) of the support structure 100. The at least one tendon guide element 130 may be made from other material, such as Teflon^{®} PTFE. The at least one tendon guide element 130 may be 3D printable onto and/or integrally formed with a core layer of the support structure 100.

The sliding mechanism comprises the sliding element 210, such as a sliding element described in connection with Figures 1 to 3. The sliding element is movably mounted on a guiding rail 220. The guiding rail 220 is arranged on and may be connected to the dorsal support portion 110. In particular, the guiding rail 220 is arranged on and may be connected (fixedly or releasably) to the surface (second surface) of the dorsal support portion 110 that is on the side opposite to the dorsal side. The guiding rail 220 defines a movement track for the sliding element 210. In the example shown in Figures 4 and 5, only one guiding rail 220 is provided. It is, however, possible to provide a plurality of guiding rails 220. This may improve the stability of the sliding motion of the sliding element 210. Instead of or in addition to the guiding rail 220, other guiding elements, such as for example guiding groove or recess, etc. may be provided. Alternatively, the guiding elements may be omitted.

The guiding rail 220 may have an elongated, substantially linear form having a substantially rectangular form (with or without rounded edges), such that flexion about only the transverse direction T (transverse axis) is possible under loading conditions.

The dimensions (width, thickness, length) of the guiding rail 220 may be suitable selected depending on the material and/or the sizes of the hand. Exemplary and non-limiting dimensions of the guiding rail 220 are width in the range of about 3 mm to 15 mm, more specifically about 4 mm to 12 mm, further specifically about 5 mm to 7 mm; length in the range of about 30 mm to 80 mm, more specifically about 40 mm to 70 mm, further specifically about 45 mm to 60 mm, thickness (height) in the range of about 0.1 mm to 1 mm, more specifically about 0.2 mm to 0.8 mm, further specifically about 0.3 mm to 0.5 mm. Exemplary guiding rail sizes are 6 x 50 x 0.4 mm or 6 x 50 x 0.3 mm. Deviations are, however, also possible depending, for example, on the intended use of the user wearable hand orthosis (e.g., for a child or adult, etc.).

The guiding rail 220 may comprise at least one portion made of a low friction material. The at least one portion made of low friction material may be the portion which engages with (i.e., which comes into contact with) the sliding element 210. This portion may be coated with a layer of low friction material or may be made of a low friction material. It is also possible to form the entire guiding rail 220 of low friction material. Non-limiting examples of low friction materials are Teflon^{®} PTFE, the iGlidur^{®} material family, Nylon 6/6, some types of hardened stainless steel, etc.

The user wearable hand orthosis may further comprise at least one support element140-x (x = 1, 2, 3, ...), configured to bear compression load. The at least support element may exhibit stiffness that is higher than that of other components or sections of the support structure 100. The at least one support element 140-x may, for example, be a low-carbon steel insert element, element of carbon fiber, fiberglass, ABS/PETG, Nylon or other materials with similar stiffness properties. The at least one support element 140-x may have a substantially strip-like form, with thickness in the range of about 0.1 mm to 0.8 mm, more specifically about 0.2 mm to 0.6 mm, further specifically about 0.2 mm to 0.4 mm; width in the range of about 3 mm to 10 mm, more specifically about 4 mm to 8 mm, further specifically about 5 mm to 7 mm; and length in the range of about 30 mm to 80 mm, more specifically in the range of about 50 mm to 95 mm, further specifically in the range of about 60 mm to 80 mm. [

In the example shown in Figures 4 and 5, there are two support elements 140-1 and 140-2 arranged on both sides of the guiding rail 220. The number, form, material and/or arrangement of the at least one support element 140-1 and 142-2 is not limited to this example and may vary.

The support elements 140-1 and 140-2 may be arranged on and connected to the surface of the dorsal support portion 110 that is on the side opposite to the dorsal side. The connection may be for example a fixed or a releasable connection. The at least one support element may also be embedded in the dorsal support portion 110, for example in a core layer of the dorsal support portion 110. The at least one support element 130 may also be an integral part of a reinforcement layer of the dorsal support portion 110. In an example, the at least one support element 130 may be provided between a core layer and the low friction layer and/or another additional layer (such as an inner or outer layer described elsewhere) of the dorsal support portion 110.

Although not shown in Figures 4 and 5, the wrist portion 125 and optionally the palmar support portion 120 may also include at least one support element, such as the support element described above, that is arranged on and connected to the surface of the respective one of the wrist portion 125 and optionally the palmar support portion 120 that is on the side opposite to the dorsal side. The connection may be for example a fixed or a releasable connection.

To realize a connection (such as a releasable connection), the support structure and in particular the at least one of the dorsal support portion 110, wrist support portion 125 and palmar support portion 120 may be provided with at least one support element connection/fastening portion 150-1 to 150-6 for connecting/fastening the at least one support element 140-1, 140-2 to the respective one of the dorsal support portion 110, wrist support portion 125 and the palmar support portion 120. Each support element connection/fastening portion 150-1 to 150-6 may be configured to receive and hold in place an end portion of a respective support element 140-1, 140-2. For example, each support element connection/fastening portion 150-1 to 150-6 may have an enclosure, into which a respective end portion of a respective support element 140-1, 140-2 is insertable. A pair of connection/fastening portions 150-1 to 150-6 may be provided for receiving and holding in place the two end portions of a respective support element 140-1, 140-2.

In the example shown in Figures 4 and 5, the dorsal support portion 110 comprises a first pair of support element connection/fastening portions 150-2 and 150-3 configured to receive and hold in place the two end portions of a first support element 140-1 and a second pair of support element connection/fastening portions of support element connection/fastening portion 150-1 and 150-4 configured to receive and hold in place the two end portions of a second support element 140-2. Further, the wrist portion 125 comprises a third pair of connection/fastening portion 150-5 and 150-6 configured to configured to receive and hold in place the two end portions of an additional support element (the additional support element is not shown in Figures 4 and 5).

The number and/or arrangement of the support element connection/fastening portions 150-1 to 150-6 not restricted to the example shown in Figures 4 and 5 may vary. For example, fewer or more support element connection/fastening portions 150-1 to 150-6 may be provided and/or the support element connection/fastening portions 150-1 to 150-6 may be arranged in a different way.

Further, the dorsal portion 110 may optionally comprise constraint elements 152-1 and 152-2 configured to constrain the movement of a respective one of the support elements 140-1 and 140-2. In particular, the support elements 140-1 and 140-2 may be elements that can flex about the transverse axis T and the provision of at least one constraint element 152-1 and 152-2 may ensure that the respective support element 140-1 and 140-2 are kept in place. Further, more than one constraint element per support element may be provided.

The user wearable hand orthosis may further comprise a hyperextension prevention mechanism configured to limit or constrain the extend of movement of the sliding element 210 in a direction toward the wrist of the user's hand, thus preventing hyperextension of the hand. The hyperextension prevention mechanism may consists of or may comprise at least one hyperextension prevention element that may, for example, be realised as a mechanical stopper element. For example, a first mechanical stopper element 160-1 may be arranged on or in the vicinity of the (optionally virtual) connection line between the dorsal support portion 110 and the wrist portion 125. Specifically, the first mechanical stopper element 160-1 may be arranged on and connected to the surface of the dorsal support portion 110 that is opposite to the dorsal surface. The first mechanical stopper element 160-1 may be arranged and configured such as to abut to a first end portion of the sliding element 210 when the sliding element 210 is in a first end position (such as shown in Figure 4), thereby restricting the movement of the sliding element 210 in the direction of the wrist of the user's hand beyond the first end position. For example, the first mechanical stopper element 160-1 may be configured and arranged such as to abut to and/or at least partially enclose a first end portion of the guiding rail 220, thereby restricting the movement of the sliding element 210 in the direction toward the wrist of the user's hand beyond the first end position.

Similarly, the movement of the sliding element 210 in a direction toward the fingers/knuckle joints may be limited by at least one second stopper element. This may, for example, be realised by employing at least one second mechanical stopper element 160-2configured to limit or constrain the extend of movement of the sliding element 210 in a direction toward the fingers of the user's hand. The at least one second mechanical stopper element 160-2may be arranged on or in the vicinity of the knuckle joints of the user's hand and may be arranged on and connected to the surface of the dorsal support portion 110 that is opposite to the dorsal surface. The at least one second mechanical stopper element 160-2may be configured to abut to a second end portion of the sliding element 210 when the sliding element is in a second end position (e.g., such as shown in Figure 5), thereby restricting the movement of the sliding element 210 in the direction toward the fingers of user's hand beyond the second end position.

For example, a second mechanical stopper element 160-2 may be configured and arranged such as to abut to and/or at least partially enclose a second end portion of the guiding rail 220, thereby restricting the movement of the sliding element 210 in the direction toward the fingers of the user's hand beyond the second end position.

The number and/or arrangement of the first and/or second stopper elements 160-1, 160-2is not restricted to the example shown in Figures 4 and 5 and may vary, e.g., additional mechanical stopper elements may be employed and/or the mechanical stopper elements may be arranged in a different way.

In addition to being configured to limit the movement of the sliding element 210, the first mechanical stopper element 160-1 and the and/or the second mechanical stopper element 160-2may be configured to receive and hold in place a guiding rail 220. In other words, the first mechanical stopper element 160-1 and/or the second mechanical stopper element 160-2may also function as a guiding rail fastening or connecting portion for connecting the guiding rail 220 to the dorsal support portion 110. The connection may be fixed or releasable connection. For example, the first mechanical stopper element 160-1 and the second mechanical stopper element 160-2 may be configured to each receive and hold in place a respective end portion of the guiding rail 220.

Thus, it is possible to realise a simple, yet efficient mechanism for limiting the movement of the sliding mechanism in a direction toward the fingers of the user's hand.

At least one of the first mechanical stopper element 160-1, the second mechanical stopper element 160-2 and the support element connection/fastening portions 150-1 to 150-6 may be formed of the same material as the dorsal support portion 110 and/or wrist portion, respectively and may be an integral part dorsal support portion 110 and/or wrist portion. It is also possible to form at least one of the first mechanical stopper element 160-1, second mechanical stopper element 160-2 and support element connection/fastening portions 150-1 to 150-6 of a different material as the dorsal support portion 110 and/or wrist portion 125.

The support structure may further comprise at least one hardware mounting element 170, configured to be engageable with one or more external hardware components. Non-limiting examples of external hardware components include one or more sensors and/or electronic equipment. The electronic equipment may for example comprise a PCB, e.g., a flexible PCB. In the example shown in Figures 4 and 5 there are a plurality of hardware mounting elements 170 formed as rounded holes on the dorsal side of the support structure. The hardware mounting elements 170 may further impart an additional stability to the support structure and thus may also have the function of support elements.

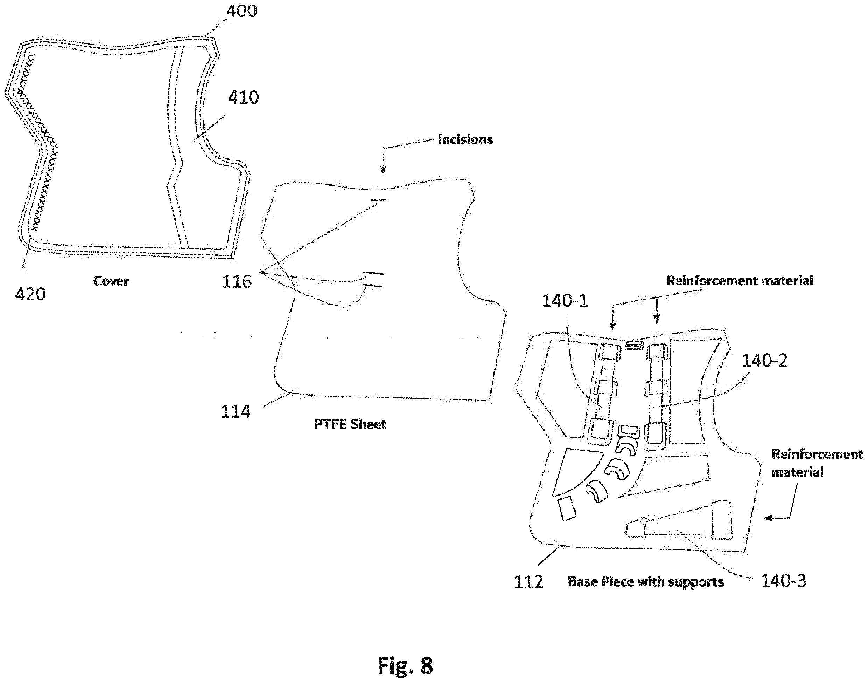

The support structure may further comprise a cover portion (not shown in Figures 4 and 5) connected or connectable to the dorsal support portion 110 (and optionally to the wrist support portion 125 and/or palmar support portion 120), wherein the cover portion is movable between an opened and a closed position. An exemplary cover portion is described below in connection with Figures 8 to 10.

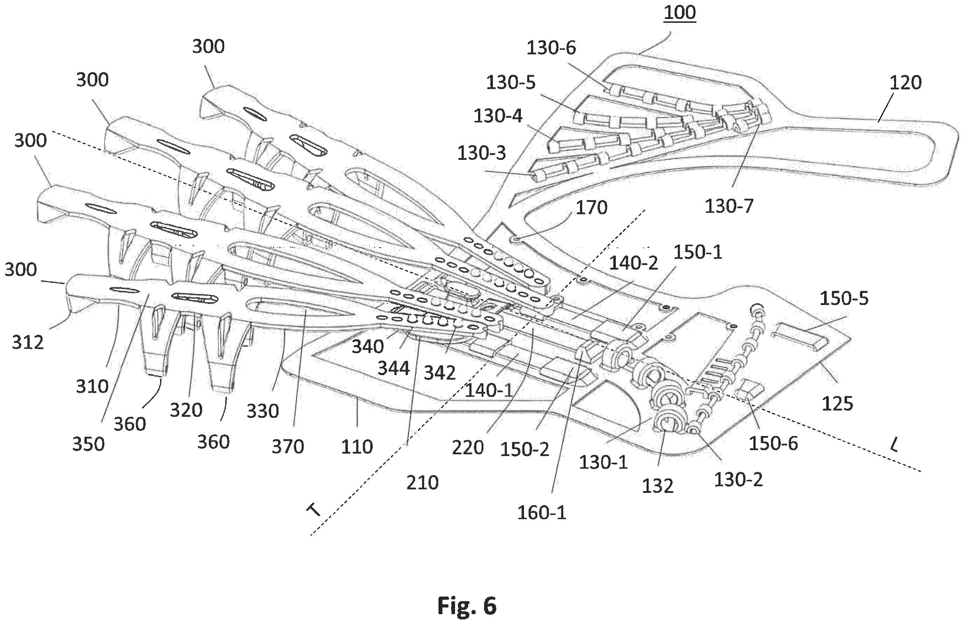

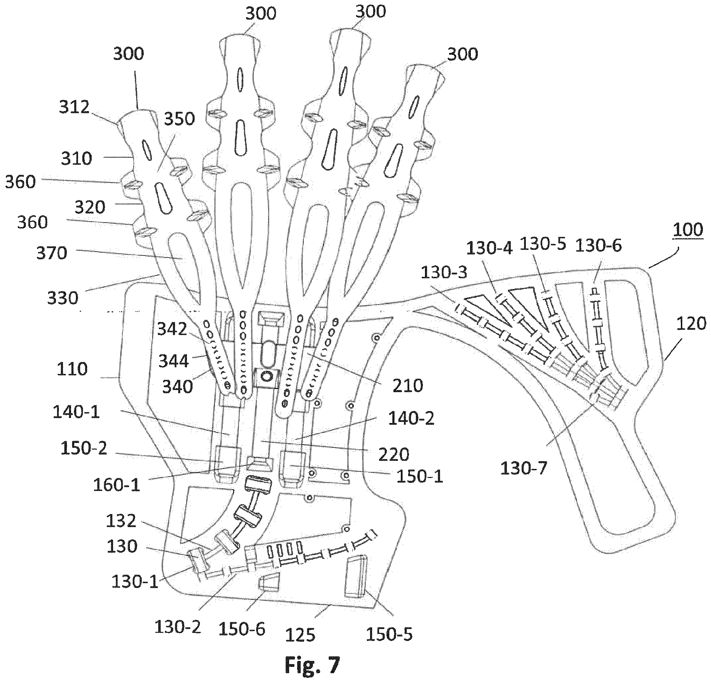

The user wearable hand orthosis may further include at least one finger module, such as the at least one finger module described above. **Figures 6** and **7** show views of an exemplary support structure 100 with a sliding mechanism mounted on it and a plurality of finger modules 300 connected to the exemplary support structure 100. The exemplary support structure 100 may be the support structure 100 described in connection with Figures 3 and 4. Specifically **Figures 6** and **7** show schematic views of the side of the support structure with mounted sliding mechanism and connected finger modules that is opposite to the dorsal side, i.e., opposite to the side facing the back of the hand and fingers of the user in a use state of the user wearable hand orthosis.

Each finger module 300 is configured to be worn on one a respective one of an index finger, a middle finger, a ring finger and a little finger of the user's hand. While the Figures show an example with four finger modules, it is possible to have fewer finger modules, such as for example only an index finger module a middle finger module and/or a ring finger module. It is also possible to configure at least one finger module to be worn on more than one finger, such as on two, three or all of the index finger, a middle finger, a ring finger, and a little finger. Further, while the Figures show an example wherein each finger module 300 is configured to be worn on one of the fingers, it is possible to configured at least one finger module to be worn on more than one of the fingers, for example on two, three or four fingers.

Each finger module 300 comprises a distal end portion (fingertip portion) that is configured to at least partially enclose a distal section, e.g., a distal phalanx section of the respective finger of the user. Specifically, the distal end portion 310 of each finger module 300 may be configured such that there is at least one contact point between the distal end portion 310 of the finger module 300 and the tip, in particular the front portion of the tip of the respective finger. The distal end portion 310 may for example be configured in form of a cap or may comprise a cap 312 that may be worn over the tip of the respective finger and at least partially encloses the at least one distal section and in particular the tip of the respective finger. Configuring the finger modules 300 to be worn on the at least distal section and in particular over a fingertip allows a more effective force transfer and response to spastic resistance.

Each of the finger modules 300 further comprise a middle portion 320 configured to be worn on a middle phalanx of the respective finger and a proximal portion 330 configured to be worn on a proximal phalanx of the of the respective finger, wherein the proximal portion 330 may be configured to extend over the knuckle joint (i.e., over a knuckle portion) of the respective finger. Further, the finger module comprises a sliding element connection portion 340.

The middle portion 320 is arranged between the distal end portion 310 and the proximal portion 330. In other words, one side of the middle portion 320 is adjacent and optionally in flush with one side of the distal end portion 310 and the other side of the middle portion 320 is adjacent and optionally in flush with one side of the proximal portion 330. The proximal portion 330 is arranged between the middle portion 320 and the sliding element connection portion 340. In other words, one side of the proximal portion 330 is adjacent and optionally in flush with one side of the middle portion 320 and the other side of the proximal portion 330 is adjacent and optionally in flush with one side of the sliding element connection portion 340. The distal end portion 310, the middle portion 320, the proximal portion 330 and the sliding element connection portion 340 may be made of the same material and form an integral structure (i.e., an integral finger module 300). It is, however, possible that at least one of the distal end portion 310, the middle portion 320, the proximal portion 330 and the sliding element connection portion 340 may be formed of or may comprise material different from that of at least one other portion. The distal end portion 310 and the sliding element connection portion 340 form the two opposite end portions of the finger module 300 in a lengthwise direction of the finger module 300.

In the example shown in the Figures, at least one of, preferentially all of the distal end portion 310, middle portion 320 and proximal portion 330 may each comprise a back portion 350 configured to substantially extend over the respective sections of the respective finger and to at least partially cover the back side of the respective finger. Further, at least one of the distal end portion 310, middle portion 320 and proximal portion 330 may further comprise at least a pair of side portions 360 extending from the back portion and configured to come into contact with side sections of the finger and/or optionally with a palmar side of the respective finger. This may improve the support of the finger modules and the force transmission to the respective fingers. Preferentially the pair of side portions are not connected on the palmar side of the finger. For example, the finger module 300 may exhibit an opening extending along the length of the finger (for example up to a finger cap) through which a finger may be inserted when mounting the finger module to the respective finger. This makes the mounting of the finger module to the respective finger of the user easier.

The back portion 350 of one or more of the distal end portion 310, middle portion 320 and proximal portion 330 may have a substantially strip form and may have a thickness in the range of about 0.1 mm to 1.5 mm, more specifically about 0.2 mm to 1.2 mm, further specifically about 0.3 mm to 1 mm. The width and/or the length of the back portion 350 of each of the distal end portion 310, middle portion 320 and proximal portion 330 may correspond to average values (not individually adjusted) or may be adjusted to fit the anatomy of the hand of an individual user. The width of the back portion 350 of the distal end portion 310, middle portion 320 and proximal portion 330 (i.e. the width of the substantially flat dorsal section of the respective finger module) may match an average or individual width of a user's finger and may for example be in the range of about 2 mm to 15 mm, more specifically in the range of 3 mm to 10 mm, further specifically about 5 mm to 8 mm. The length of the back portion 350 of the distal end portion 310, middle portion 320 and proximal portion 330 (i.e. the length of the substantially flat dorsal section of the respective finger module) may match an average or individual length of a user's finger and may for example be in the range of about 20 mm to 110 mm. more specifically about 25 to 100 mm, further specifically about 30 to 90 mm, depending on the intended use (e.g. for an adult, child, specific finger, etc.).

The back portion 350 of one or more of the distal end portion 310, middle portion 320 and proximal portion 330 may exhibit one or more slits or openings. For example, the back portion 250 of the proximal portion 330 may have at least one opening 370 (formed as a though hole in the back portion) in a middle section of the back portion 350, wherein the at least one opening 370 extends in a lengthwise direction of the respective finger module 300. The opening 370 may be configured and arranged such that when the finger module is in a use state, the opening 370 is positioned substantially above the knuckle joint (i.e., over a knuckle portion) of the respective finger. This may reduce the compression force applied on the knuckle joint and may improve the wearability of the user wearable hand orthosis.

The back portion(s) 350 and/or the pair of side portions 360 may be made of an elastic polymer, thermoplastic polyurethane (TPU), thermoplastic polymers (TPE), etc.

The sliding element connection portion 340 is arranged adjacent the proximal portion 330 and may form an end portion of the respective finger module 300. The sliding element connection portion 340 may comprise one or more connection elements 342 that are engageable with one or more connection elements of a respective finger module connection portion 218-x of the sliding element 210, such as to realize a releasable rigid connection. For example, the sliding element connection portion 340 may comprise a plurality of connection elements 342 that are arranged substantially in a line along a lengthwise direction of the sliding element connection portion 340 and thus along a lengthwise direction of the respective finger module 300. By changing the pairing between a connection element 342 of the sliding element connection portion 340 of the finger module 300 and a connection element 219 of a respective finger module connection portion 218-x of the sliding element 210, it is possible to realize length adjustment, based for example on the size of the hand of the user.

In the example shown in the Figures, the sliding element connection portion 340 comprises a body portion 344 having a substantially strip-like or plate-like form. The thickness of the body portion 344 may be for example in the range of about 1 mm to 7 mm, more specifically about 2 mm to 6 mm, further specifically about 3 mm to 5 mm. The width of the body portion 344 may be for example in the range of about 2 mm to 10 mm, more specifically about 3 mm to 8 mm, further specifically about 4 mm to 7 mm. and length in the range of about 5 to 35 mm, more specifically about 6 to 30 mm, further specifically about 8 to 25 mm. One end of the body portion 342 is connected to and is optionally in flush with one end portion of the proximal portion 330. Each connection element 342 is in form of a through opening provided in the body portion 344, which is engageable with one or more of the connection elements (e.g., pegs) 219 of the respective finger module connection portion 218 provided on the sliding element 210.

However, the present description is not limited to this example and other types and/or numbers and/or arrangements of connection elements 342 on the side of the finger module and respective connection elements 219 on the side of the sliding element may be employed.

The user wearable hand orthosis may further comprise a thumb module, not shown in the Figures. The thumb module may be a thumb module as described above.

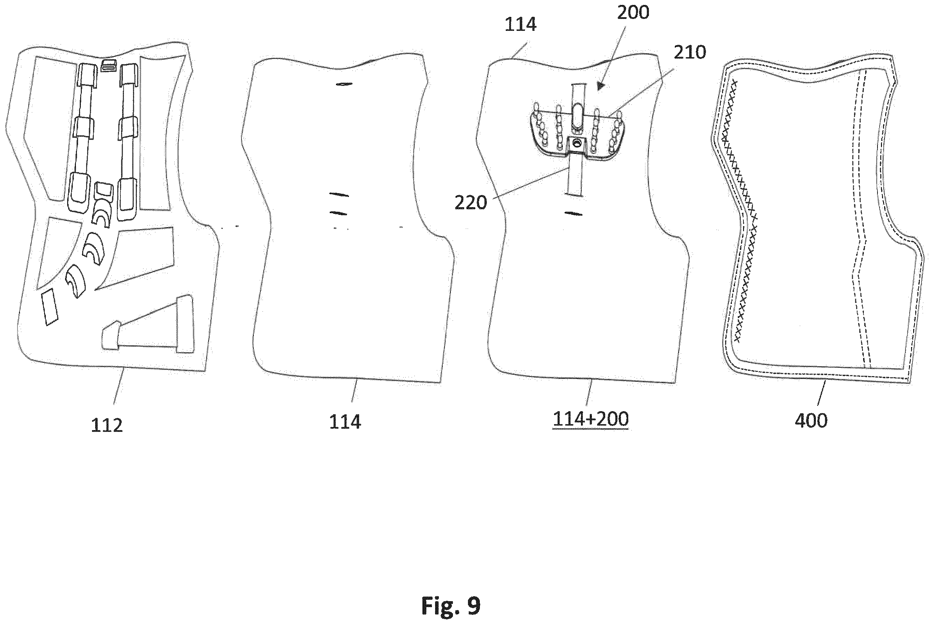

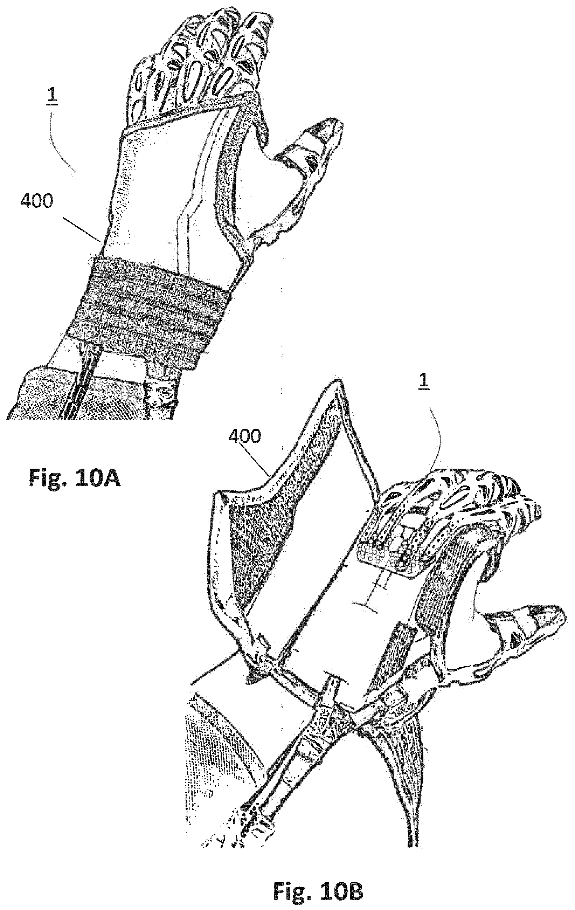

**Figures 8** and **10** show components of an exemplary support structure for user wearable hand orthosis, wherein a sliding mechanism is mountable on the support structure. The exemplary support structure and sliding mechanism may be the one described in connection with Figures 1 to 7. **Figure 8** shows a schematic top view of components of an exemplary support structure prior to mounting a sliding mechanism 200 comprising a sliding element 210 and a guiding rail 220, **Figure 9** shows a schematic top view of components of an exemplary support structure according to Figure 8, wherein the sliding mechanism 200 is being mounted on the support structure and more specifically on the dorsal support portion 110 of the support structure. Further, Figure 9 shows from left to right the assembly steps of the components.

As shown in Figures 8 and 9 the support structure includes a dorsal support portion and a wrist support portion which are integrally formed as one piece. Further, the support structure comprises a cover portion 440. The support structure may include further components as described in connection with Figures 1 to 7. For better visibility, the further components are not shown in Figures 8 and 9.

The dorsal support portion 110 and the wrist support portion 125 may be the one described in connection with Figures 1 to 7, a reference to which is made. In the example shown in Figures 8 and 9, the piece formed by the dorsal support portion and the wrist support portion has a multilayer structure comprising a core layer 112 (which may also function as a reinforcement layer) and a low friction layer 114 arrangeable on the side of the core layer 112 opposite to the side facing the hand of the user.

The core layer 112 may be a core layer as described above and may be configured to provide stability of the support structure. The core layer 112 may be formed of at least one metal (e.g., low carbon steel), at least one elastomer, at least one polymer (e.g., silicone, thermoplastic urethane (TPU), thermoplastic elastomer (TPE), and/or PA-6 (polyamide 6)), and/or at least one non-textile material. As described above, the core layer 112 may itself a plurality of layers. Alternatively or in addition, the core layer may comprise a plurality of sections with different properties, as described above.

Further, the core layer 112 may be configured to form at least one of the portions and/or elements described in connections with Figures 1 to 7. Non limiting examples include one or more of tendon guiding groove, guiding rail connection/fastening portion, support element connection/fastening portion, hyperextension prevention element, stopper elements, hardware mounting element(s), locking section(s), etc.. Further, three supporting elements 140-1 to 140-3 of reinforcement material are provided on a surface of the core layer 112 opposite to the surface facing the back of the hand. Regarding the description of each of the portions and/or elements, a reference is made to Figures 1 to 7 and the related description.

The low friction layer 114 may be a layer made of Teflon° PTFE, Nylon woven fabric, silk-blended fabrics, Lycra/spandex blended fabrics or any other suitable material. The low friction layer may comprise a plurality of incisions for inserting the at least one guiding rail 220 such as the low friction layer 114 is positioned between the core layer 112 and the guiding rail 220 (such as for example shown in Figure 9 (see element "114 + 200"). Thus, the movement of the sliding element 210 mounted on the guiding rail 220 may be facilitated. The low friction layer may comprise additional incisions, for example for inserting a tendon or other force transfer element.

The cover portion 400 may be the cover portion described above. In particular, in a closed position of the cover portion 400 a first surface of the cover portion 400 arranged on the dorsal side (i.e., the surface facing the second surface of the dorsal support portion 110 and/or wrist portion) may cover the sliding mechanism 200 with the sliding element 210 and the guiding rail 220 and may optionally cover a connection portion of the finger modules 300 connected to the sliding element 210. The cover portion 400 thus may constrain the sliding element 210 and/or the finger modules 300 as described above. The cover portion 400 may be configured as a soft, folding cover. The cover portion 400 may have a locking section 410 configured to engage with a corresponding locking section on one or more of the dorsal support portion 110, palmar support portion 120 and wrist portion 125, so as to fix the cover portion 400 in place when the dorsal support portion 400 is in a closed position. The cover portion 400 may further comprise a connection section 420 for connecting the cover portion 400 to one or more of the dorsal support portion 110, palmar support portion 120 and wrist portion 125, wherein the connection is configured such that the cover portion 400 is rotatably movable between an open and a closed position.

**Figures 10A** and **10B** show an exemplary user wearable hand orthosis 1 in a use state when the user wearable hand orthosis 1 is mounted on the hand of the user, wherein Figure 10A shows the exemplary user wearable hand orthosis 1 in a state where the cover portion 400 is in a closed position and Figure 10B shows the exemplary user wearable hand orthosis 1 in a state where the cover portion 400 is in a opened position. The user wearable hand orthosis 1 may be a user wearable hand orthosis 1 as described in connection with Figures 1 to 9. In particular, the user wearable hand orthosis may be a tendon-driven glove orthosis configured to be worn on a hand of a user.

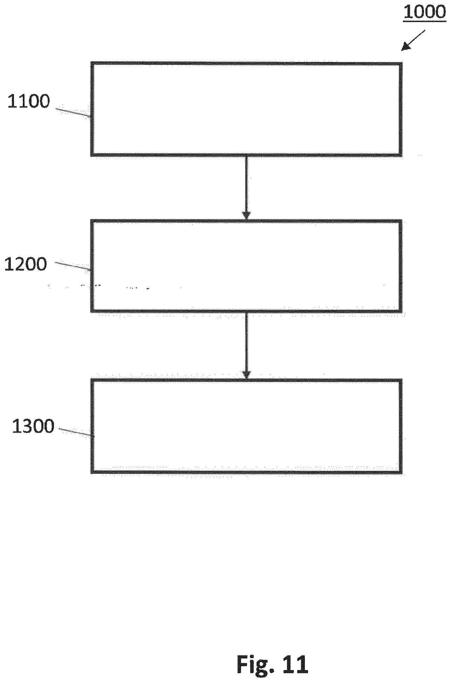

**Figure 11** shows an exemplary flow chart of a method 1000 of manufacturing a user wearable hand orthosis. The user wearable hand orthosis may comprise a support structure 100 configured to be worn on a hand of a user, a sliding mechanism and at least one finger module 300 as described for example in connection with Figures 1 to 10.

In a first step 1100 a support structure is provided. The support structure may be a support structure 100 as described for example in connection with Figures 1 to 10. In particular, the support structure 100 may comprise a dorsal support portion 110 configured to be worn on at least a part of a back of the hand of the user, wherein the dorsal support portion 110 has a first surface on a dorsal side of the dorsal support portion 210 and a second surface on a side of the dorsal support portion 210 opposite to the dorsal side.

In a second step 1200 a sliding mechanism is provided. The sliding mechanism may be the sliding mechanism as described in connection with Figures 1 to 10. In particular, the sliding mechanism may comprise a sliding element 210 configured to move along a track on the second surface of the dorsal support portion 210 between a first end position and a second end position. In a use state of the user wearable hand orthosis, the first end position is arranged proximal to a wrist portion of the hand of the user and the second end position is arranged at a distance from the first end position in a direction toward a knuckle joint portion of the hand of the user (and thus in a direction toward the finger of the user's hand).

In a third step 1300 at least one finger module configured to be worn on at least one finger of the hand of the user is provided. The at least one finger module may be at least one finger module 300 as described in connection with Figures 1 to 7. In particular, the at least one finger module may comprise a distal end portion 310 configured to be worn on at least one distal section of the at least one finger and a proximal portion 340 connected or connectable to the sliding element 210.

The steps 1100, 1200 and 1300 may be executed in a different order.

The providing of the support structure and/or sliding mechanism and/or at least one finger module may include selecting and/or retrieving a premanufactured support structure and/or sliding mechanism and/or at least one finger module from a storage. Alternatively or in addition, the providing of a support structure and/or sliding mechanism and/or at least one finger module may include manufacturing of the support structure and/or the sliding mechanism and/or the at least one finger module, for example by using known methods, such as additive methods, 3D printing, moulding (such as injection moulding), knitting, etc.

Providing of the support structure may comprise providing a multilayer structure such as the one described above in connection with the user wearable hand orthosis. In particular, providing of the support structure may comprise:
providing a core layer; and/or
providing at least one additional layer, wherein the at least one additional layer may be at least one low friction layer arranged on the dorsal side and/or on the side opposite to the dorsal side of the core layer.

The method may comprise further steps such as for example one or more of the following steps:
- connecting the sliding mechanism to the dorsal support portion 210, wherein the connecting of the sliding mechanism to the dorsal support portion 210 may optionally include movably mounting the sliding element 210 on a guiding rail 220 arranged on a surface of the dorsal support portion 110 that is opposite to a dorsal surface of the dorsal support portion 110;
- connecting at least one proximal portion of the at least one the finger module 300 to the sliding element 210;
- adjusting the length from a connection portion (anchor point or portion) of the at least one finger module 300 to the distal end portion of the at least one finger module 300;
- connecting at least one support element 140-x and/or at least one guiding rail 220 to the dorsal support portion 110;
- connecting the sliding element 210 to at least one force transfer element (e.g., tendon) configured to exert a (pulling) force on the sliding element 210;
- moving the cover portion 400 into closed position;
- wrapping the support structure 110 substantially around the hand to bring a first locking section of the support structure 110 into engagement with a second locking section of the support structure 110 to mount the support structure on the hand of the user.

The above steps may be executed in a different order.

As readily understood from the present disclosure, while the invention has been described by means of exemplary embodiments illustrated in the appended Figures, the invention is not limited thereto. Instead, any combination of features described herein and/or shown in the appended Figures may be implemented.

### List of Reference Numerals

- 1: user wearable hand orthosis
- 100: support structure
- 110: dorsal support portion
- 112: core layer
- 114: low friction layer
- 116: incision
- 120: palmar support portion
- 125: wrist portion
- 130: tendon guide element (exemplary force transfer element guide element)
- 130-1 to 130-7: groups of tendon guide elements
- 132: tendon groove
- 140-1 to 140-3: support elements (reinforcement elements)
- 150-1 to 150-6: support element connection/fastening portions
- 152-1, 152-2: constrain elements for the support elements
- 160-1: first mechanical stopper element
- 160-2: second mechanical stopper element
- 170: hardware mounting element(s)
- 200: sliding mechanism
- 210: sliding element
- 212: body portion of the sliding element
- 212-1, 212-2: major surfaces of the body portion of the sliding element;
- 212-3: peripheral surface of the body portion of the sliding element
- 214: tendon connection portion (exemplary force transfer element connection portion)
- 214-2: intermediate portion of the tendon connection portion
- 214-3: head portion of the tendon connection portion
- 214-5: through hole
- 215: guiding rail engagement portion
- 215-2: side walls of the guiding rail engagement portion
- 215-4: bottom plate of the guiding rail engagement portion
- 215-6: guiding rail accommodation space
- 216: motion extension portion
- 216-2: opening of the motion extension portion
- 216-4: side walls of the motion extension portion
- 216-6: top plate of the motion extension portion
- 216-8: opening for a tendon passage
- 218-1 to 218-4: finger module connection portions
- 219: connection element(s)
- 220: guiding rail
- 300: finger module
- 310: distal end portion of the finger module
- 312: finger tip cap of the finger module
- 320: middle portion of the finger module
- 330: proximal portion of the finger module
- 340: sliding element connection portion of the finger module
- 342: connection elements
- 344: body portion
- 350: back portion
- 360: a pair of side portion
- 370: opening
- 400: cover portion
- 410: locking section of the cover portion
- 420: connection section of the cover portion
- 500: tendon (exemplary force transfer element)
- 1000: method
- 1100, 1200, 1300: method steps
- L: Longitudinal direction (Length axis)
- T: Transverse direction (Transverse axis)

## Claims

1. A user wearable hand orthosis (1) comprising;
a support structure (100) comprising a dorsal support portion (110) configured to be worn on at least a part of a back of the hand of the user,
wherein the dorsal support portion (110) has a first surface on a dorsal side of the dorsal support portion (110) and a second surface on a side of the dorsal support portion (110) opposite to the dorsal side;
a sliding mechanism (200) comprising a sliding element (210) configured to be movable along a track on the second surface side of the dorsal support portion (110) between a first end position and a second end position, wherein in a use state of the hand orthosis (1) the first end position is arranged proximal to a wrist portion of the hand of the user and the second end position is arranged at a distance from the first end position in a direction toward a knuckle joint portion of the hand of the user; and
at least one finger module (300) configured to be worn on at least one finger of the hand of the user, wherein each of the at least one finger modules (300) comprises a distal end portion (310), configured to be worn on at least one distal section of the at least one finger, and a sliding element connection portion (340), configured to be connectable to the sliding element (210).

2. The user wearable hand orthosis (1) according to claim 1, further comprising at least one force transfer element (500) connected or connectable to the sliding element (210) and configured to exert a force on the sliding element (210), such as to enable the movement of the sliding element (210) between the first end position and the second end position.

3. The user wearable hand orthosis (1) according to claim 1 or 2, wherein the sliding mechanism (200) further comprises at least one guiding rail (220) and/or guiding recess arranged on the second surface of the dorsal support portion (110), wherein the sliding element (210) is movably mounted or mountable on the at least one guiding rail (220) and/or guiding recess.

4. The user wearable hand orthosis (1) according to claim 3,
wherein the guiding rail (220) and/or guiding recess comprises at least one portion made of a low friction material.

5. The user wearable hand orthosis (1) according to any one of the preceding claims, wherein the sliding element (210) comprises a body portion (212) having a substantially plate-like form.

6. The user wearable hand orthosis (1) according to any one of the preceding claims, wherein the support portion (110) comprises
a core layer (112); and/or
at least one low friction layer (114) arranged on the side facing the hand of the user and/or on the side of the support portion (110) opposite to the side facing the hand of the user.

7. The user wearable hand orthosis (1) according to any one of the preceding claims, wherein the support structure (100) further comprises a cover portion (400) connected or connectable to the dorsal support portion (110), wherein the cover portion (400) is movable between an opened position and a closed position, wherein in the closed position a first surface of the cover portion (400) arranged on the dorsal side covers the sliding element (210).

8. The user wearable hand orthosis (1) according to claim 7, wherein the cover portion (400) comprises a low friction layer arranged on the dorsal side.

9. The user wearable hand orthosis (1) according to any one of the preceding claims, further comprising
at least one support element (140-1, 140-2, 140-3), and/or
at least one hyperextension prevention mechanism (150-1, 150-2, 150-3).

10. A method of manufacturing a user wearable hand orthosis (1) configured to be worn on a hand of a user, said method comprising:
providing a support structure (100) comprising a dorsal support portion (110) configured to be worn on at least a part of a back of the hand of the user, wherein the dorsal support portion (110) has a first surface on a dorsal side of the dorsal support portion (110) and a second surface on a side of the dorsal support portion (110) opposite to the dorsal side;
providing a sliding mechanism (200) comprising a sliding element (210) configured to be movable along a track on the second surface of the dorsal support portion (110) between a first end position and a second end position, wherein in a use state of the user wearable hand orthosis (1) the first end position is arranged proximal to a wrist portion of the hand of the user and the second end position is arranged at a distance from the first end position in a direction toward a knuckle joint portion of the hand of the user;
providing at least one finger module (300) configured to be worn on at least one finger of the hand of the user, wherein each of the at least one finger modules (300) comprises a distal end portion (310), configured to be worn on at least one distal section of the at least one finger, and a sliding element connection portion (340) configured to be connectable to the sliding element (210).

11. The method (100) according to claim 10, further comprising connecting the sliding mechanism (200) to the dorsal support portion (110),
wherein the connecting of the sliding mechanism (200) to the dorsal support portion (110) optionally comprises movably mounting the sliding element (210) on at least one guiding rail (220) arranged on a surface of the dorsal support portion (110) that is opposite to a dorsal surface of the dorsal support portion (110).

12. The method (1000) according to claim 10 or 11, further comprising connecting at least one proximal portion of the at least one the finger module (300) to the sliding element (210),
wherein the connecting the at least one sliding element connection portion (340) of the at least one the finger module (300) to the sliding element (210) optionally comprises adjusting the length from a connection portion of the at least one finger module (300) to the distal end portion of the at least one finger module (300).

13. The method according to one of claims 10 to 12, further comprising connecting the sliding element (210) to at least one force transfer element (500) configured to exert a force on the sliding element (210).

14. The method according to one of claims 10 to 13, wherein providing the support structure (100) comprises providing:
a core layer (112); and/or
at least one low friction layer (114) arranged on the dorsal side and/or on the side opposite to the dorsal side of the core layer.

15. The method according to any one of claims 10 to 14, further comprising:
wrapping the support structure (100) substantially around the hand to bring a first locking section of the support structure (100) into engagement with a second locking section of the support structure (100) to mount the support structure on the hand of the user.
